# EUROPEAN PATENT SPECIFICATION

(11) **EP 2 235 163 B1**
(45) Date of publication and mention of the grant of the patent: **08.01.2014**
(21) Application number: 08853526.5
(22) Date of filing: 26.11.2008
(51) Int. Cl.: C12N 7/00, A61K 39/00, A61K 39/04, C07K 14/195, C12N 15/52

(54) **RECOMBINANT BCG TUBERCULOSIS VACCINE FOR ELICITING IMMUNE RESPONSES TO MYCOBACTERIUM TUBERCULOSIS**
REKOMBINANTER BCG-TUBERKULOSE-IMPFSTOFF ZUM HERVORRUFEN VON IMMUNREAKTIONEN GEGEN MYCOBACTERIUM TUBERCULOSIS
VACCIN BCG RECOMBINANT CONTRE LA TUBERCULOSE POUR INDUIRE DES RÉPONSES IMMUNITAIRES CONTRE MYCOBACTERIUM TUBERCULOSIS

(30) Priority: 27.11.2007 US 945680
(43) Date of publication of application: 06.10.2010
(73) Proprietor: Aeras Global TB Vaccine Foundation, Rockville, MD 20850 (US)
(72) Inventor: SHAFFERMAN, Avigdor, 74037 Ness Ziona (IL); ZVI, Anat, 70700 Gedera (IL); ARIEL, Naomi, 74041 Ness Ziona (IL); FULKERSON, John, Silver Spring MD 20901 (US); SUN, Ronggai, Ellicott City MD 21042 (US); CHANG, Rosemary, Gaithersburg MD 20878 (US); SADOFF, Jerald, C., Washington DC 20012 (US)
(74) Representative: Kröncke, Rolf
(86) International application number: PCT/US2008/084904
(87) International publication number: WO 2009/070700

(56) References cited:
- US-A- 5 854 055
- US-A1- 2004 057 963
- US-A1- 2007 128 216
- HARTH GUNTER ET AL: "A two-plasmid system for stable, selective-pressure-independent expression of multiple extracellular proteins in mycobacteria", MICROBIOLOGY, SOCIETY FOR GENERAL MICROBIOLOGY, READING, GB, vol. 150, no. Part 7, 1 July 2004 (2004-07-01), pages 2143-2151, XP002445319, ISSN: 1350-0872, DOI: 10.1099/MIC.0.27113-0
- ROSENKRANDS I ET AL: "Hypoxic response of Mycobacterium tuberculosis studied by metabolic labeling and proteome analysis of cellular and extracellular proteins", JOURNAL OF BACTERIOLOGY, AMERICAN SOCIETY FOR MICROBIOLOGY, WASHINGTON, DC; US, vol. 184, no. 13, 1 July 2002 (2002-07-01) , pages 3485-3491, XP002260866, ISSN: 0021-9193, DOI: 10.1128/JB.184.13.3485-3491.2002
- DHAR NEERAJ ET AL: "Immunogenicity of recombinant BCG vaccine strains overexpressing components of the antigen 85 complex of Mycobacterium tuberculosis.", MEDICAL MICROBIOLOGY AND IMMUNOLOGY, vol. 193, no. 1, February 2004 (2004-02), pages 19-25, XP002677664, ISSN: 0300-8584
- COLE ET AL.: 'Deciphering the biology of Mycobacterium tuberculosis from the complete genome sequence' NATURE, [Online] vol. 393, 1998, pages 537 - 544 Retrieved from the Internet: <URL:http://genolist.pasteur.fr/TubercuList /genome.cgi>
- CAMUS ET AL.: 'Re-annotation of the genome sequence of Mycobacterium tuberculosis H37Rv' MICROBIOLOGY, [Online] vol. 148, 2002, pages 2967 - 2973 Retrieved from the Internet: <URL:http://genolist.pasteur.fr/TubercuList /genome.cgi>

## Description

### BACKGROUND OF THE INVENTION

### Field of the Invention

The invention generally relates to improved *Mycobacterium tuberculosis* (Mtb) vaccines that are successful in preventing the development of symptoms of tuberculosis, both pre- and post-exposure to Mtb. In particular, the invention provides an improved recombinant Bacille Calmette-Guerin (BCG) subunit-based vaccine in which one or more Mtb antigens and one or more Mtb resuscitation or reactivation antigens are overexpressed, and in which at least a portion of the DosR regulon is up-regulated.

### Background of the Invention

The current prophylactic (pre-exposure) *Mycobacterium tuberculosis* (Mtb) vaccine *Mycobacterium bovis* (*M. bovis*) BCG, introduced over 60 years ago, efficiently protects against severe disease manifestation in children but fails to prevent the establishment of latent TB or disease reactivation of infection in adolescents and adults. Moreover, essentially all novel Mtb vaccines currently in clinical trials are designed as prophylactic rather than both prophylactic and therapeutic (post exposure) vaccines.

It is believed that Mtb progresses through a series of stages during its infectious cycle in man as a reaction to human immune responses and that each stage is orchestrated by a distinct genetic program which directs the expression of stage-specific antigens. If this concept is valid, then a truly comprehensive tuberculosis vaccine should include antigens representing each stage as well as antigens that are stage-independent. Latent tuberculosis (LTBI/latency) appears to be one such stage and current evidence suggests that Mtb adopts a unique physiological phenotype during latency characterized by bacteriostasis (non-replicating persistence), a switch from aerobic to anaerobic respiration, expression of the α-crystalline small chaperone protein (Acr/HspX) and increased resistance to several mycobacterial antibiotics.

Maintenance of the non-replicating persistence state, believed to be typical of Mtb in latent lesions, appears to depend on the continuous production of Th1 cytokines (IFNγ, IL-12 and TNFα) and nitric oxide and the localization of MTB within stable granulomas. However, the reactivation of latent Mtb infection, characterized by resumption of bacterial replication, inflammation and cavitation, can be promptly precipitated by immunosuppressive regimens (e.g., corticosterioids or TNFα-antagonist) and occurs in 5-10% of latently infected individuals, perhaps due to acquired tolerance to environmental mycobacteria, age, and, more significantly, HIV disease. This common clinical scenario and the proven role of the cellular immune system for the maintenance of latency lead to the conclusion that non-replicating persistence is a metastable phenotype determined by three interacting processes: bacterial replication within latent lesions is constrained by effectors of the cellular immune system; bacteria within latent lesions monitor the production of immune effectors; and, decreased production of immune effectors results in resumption of replication.

To date, no successful vaccines have been developed which confer immunity to infection by Mtb and at the same time treat or prevent the development of symptoms of TB after exposure to Mtb, or as a result of reactivation of latent infection. A recombinant BCG vaccine, engineered to elicit an immune response of this kind, might reduce reactivation rates in persons with subtle degrees of immunosuppression produced, for example, by senescence, diabetes, HIV disease, acquired tolerance to environmental mycobacteria or malnutrition. There is thus an ongoing need to develop new TB vaccines, and it would be particularly useful to develop a vaccine that can be used both prophylactically and for post-exposure treatment.

### SUMMARY OF THE INVENTION

The present invention is based on the development of a novel recombinant Bacille Calmette-Guerin (rBCG) for use as a vaccine. The vaccine may be used prophylactically to prevent Mtb infection in naive individuals. However, the vaccine is also effective for treating individuals who have already been exposed to and/or infected by Mtb. The vaccine prevents the establishment of infection and likewise prevents the reactivation of latent Mtb in individuals that have been previously infected. The rBCG that is used in the vaccine preparations is genetically engineered to express "classical" Mtb antigens and antigens that are relevant to several stages of the Mtb life cycle, e.g. latency, reactivation and resuscitation. Thus, the immune response that is generated as a result of immunization with the vaccine protects the vaccinated individual from developing an active Mtb infection at any and all stages of exposure to Mtb. In particular, the rBCG overexpresses 1) one or more genes encoding Mycobacterium tuberculosis (Mtb) antigens that are known to elicit potent, protective immune responses to Mtb; and 2) one or more genes encoding at least one Mtb resuscitation or reactivation antigen. The antigen encoding sequences are located on an extrachromosomal element or are integrated into the chromosome of the recombinant BCG. In addition, expression of all or part of the Dos R regulon is up-regulated in the novel rBCG.

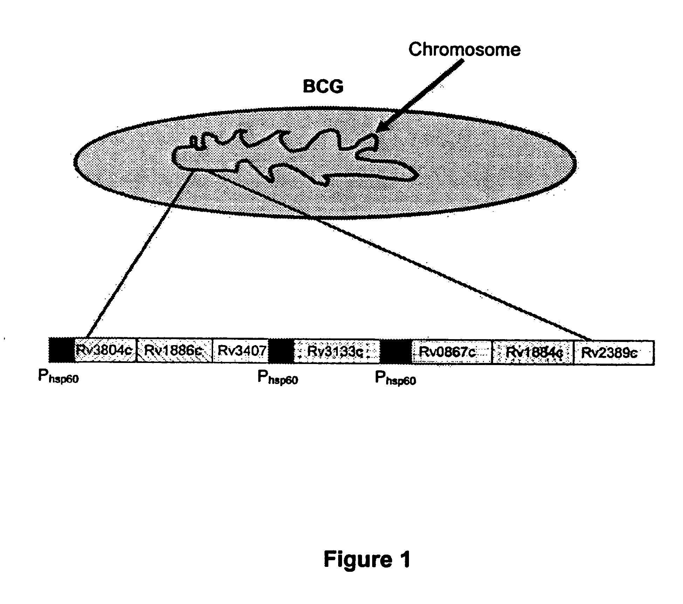

The embodiment of the invention in which the antigen encoding sequences are integrated into the chromosome is depicted schematically in Figure 1.

### BRIEF DESCRIPTION OF THE DRAWINGS

Figure 1. Schematic representation of the rBCG of the invention.
Figure 2. Diagram of integration plasmid and expression cassette.
Figure 3A-D. Immunoblots demonstrating the expression of chromosomally integrated antigen cassettes. A, Expression or Rv0867c from AERAS-407 precursor AFV-102pRC108, Lane 1 = AFV-102pRC108, Lane 2 = AFV-102; B, Overexpression of Ag85A and Ag85B from AERAS-407 precursor AFV-102RC108, Lane 1 = AFV-102, Lane 2 = AFV-102pRC108; C, Expression of Rv3407 from AERAS-407 precursor AFV-102pRC108, Lane 1 = AFV-102, Lane 2 = AFV-102pRC108; D, Expression of DosR (Rv3133c) from AERAS-407 precursor AFV-102pRC108, Lane 1 = AFV-102, Lane 2 = AFV-102pRC108.
Figure 4. Diagram of a second integration plasmid and expression cassette.
Figure 5A and B. Rv3804c amino acid sequence (A, SEQ ID NO: 1) and nucleotide sequence (B, SEQ ID NO: 2).
Figure 6A and B. Rv1886c amino acid sequence (A, SEQ ID NO: 3) and nucleotide sequence (B, SEQ ID NO: 4).
Figure 7A and B. Rv0867c amino acid sequence (A, SEQ ID NO: 5) and nucleotide sequence (B, SEQ ID NO: 6).
Figure 8A and B. Rv1009c amino acid sequence (A, SEQ ID NO: 7) and nucleotide sequence (B, SEQ ID NO: 8).
Figure 9A and B. Rv1884c amino acid sequence (A, SEQ ID NO: 9) and nucleotide sequence (B, SEQ ID NO: 10).
Figure 10A and B. Rv2389c amino acid sequence (A, SEQ ID NO: 11) and nucleotide sequence (B, SEQ ID NO: 12).
Figure 11A and B. Rv2450c amino acid sequence (A, SEQ ID NO: 13) and nucleotide sequence (B, SEQ ID NO: 14).
Figure 12A and B. Rv2623c amino acid sequence (A, SEQ ID NO: 15) and nucleotide sequence (B, SEQ ID NO: 16).
Figure 13A and B. Rv0288c amino acid sequence (A, SEQ ID NO: 17) and nucleotide sequence (B, SEQ ID NO: 18).
Figure 14A and B. Rv2626c amino acid sequence (A, SEQ ID NO: 19) and nucleotide sequence (B, SEQ ID NO: 20).
Figure 15A and B. Rv2005c amino acid sequence (A, SEQ ID NO: 21) and nucleotide sequence (B, SEQ ID NO: 22).
Figure 16A and B. Rv1996c amino acid sequence (A, SEQ ID NO: 23) and nucleotide sequence (B, SEQ ID NO: 24).
Figure 17A and B. Rv0685c amino acid sequence (A, SEQ ID NO: 25) and nucleotide sequence (B, SEQ ID NO: 26).
Figure 18A and B. Rv0824c amino acid sequence (A, SEQ ID NO: 27) and nucleotide sequence (B, SEQ ID NO: 28).
Figure 19A and B. Rv2029c amino acid sequence (A, SEQ ID NO: 29) and nucleotide sequence (B, SEQ ID NO: 30).
Figure 20A and B. Rv2627c amino acid sequence (A, SEQ ID NO: 31) and nucleotide sequence (B, SEQ ID NO: 32).
Figure 21A and B. Rv2744c amino acid sequence (A, SEQ ID NO: 33) and nucleotide sequence (B, SEQ ID NO: 34).
Figure 22A and B. Rv3347c amino acid sequence (A, SEQ ID NO: 35) and nucleotide sequence (B, SEQ ID NO: 36).
Figure 23A and B. Rv1130c amino acid sequence (A, SEQ ID NO: 37) and nucleotide sequence (B, SEQ ID NO: 38).
Figure 24A and B. Rv1169c amino acid sequence (A, SEQ ID NO: 39) and nucleotide sequence (B, SEQ ID NO: 40).

### DETAILED DESCRIPTION OF THE PREFERRED

### EMBODIMENTS OF THE INVENTION

The present invention provides a novel rBCG for use in Mtb vaccine preparations. The rBCG is genetically engineered to overexpress 1) one or more Mtb antigens, which may include so-called "classical" Mtb antigens such as Rv1886c-Ag85B ("85B") and Rv3804c-Ag85A ("85A"), among others; and 2) at least one Mtb resuscitation/reactivation antigen. In addition, in the rBCG of the invention, the DosR regulon, or a portion thereof, is up-regulated. The DosR regulon, comprised of 48 genes, is typically activated in Mtb as a result of a post-infection drop in 02 tension (hypoxia).

The Mtb antigens that are selected for inclusion in the rBCG of the invention are generally those which are known or predicted to elicit a protective immune response in individuals exposed to the antigens. Various criteria may be used to select suitable antigens, including but not limited to: the observation that individuals with immune responses to the antigen are able to control Mtb infection, determination that the antigen contains one or more T-cell epitopes via an immunoinformatics analysis (e.g. using a program such as that which is found at the website of the Technical University of Denmark: cbs.dtu.dk/services/NetCTL, in which the analysis is based on the identification of proteosome cleavage sites, endoplasmic reticulum (ER) transport efficiency, major histocompatibility (MHC) class 1 binding affinity, etc.); an analysis of experimental evidence based on an in-depth literature search; sorting or ranking of results according to various parameters such as macrophage survival/persistence; up-regulation of expression by the two-component system MprAB; response to hypoxia; involvement in dormancy; expression in lung tissue; genes with promoters that are co-regulated with Rv2031 (Acr); proteins that are secreted (which are more accessible to the immune system); presence of repeats (many virulence associated proteins have amino acid repeat domains); ability to act as a B-cell immunogen; cell wall associated or cell walls biogenesis (membrane-exposed/associated proteins are considered to be more accessible to the immune system; existing vaccine efficacy data; uniqueness to Mtb; etc.) Thus, antigens may be selected based on experimental data demonstrating their efficacy, or alternatively (or in addition) such antigens may be selected based on their predicted abilities. Those of skill in the art are familiar with the implementation of such analyses, and with developing scoring or ranking systems in order to assign a weighted comparative score to candidate antigens. For example, numeric scores may be assigned for each attribute under consideration, and the antigens with the highest cumulative totals may be selected for use. In addition, other considerations may play a role in the decision making process, some of which are practical in nature (e.g. the availability of the antigen, the ease of expressing the antigen, the ease of measuring immune responses to the antigen, etc.)

The starting pool of antigens may be any or all known Mtb open reading frame (ORF) products. Those of skill in the art are familiar with sources for identifying Mtb ORF products, e.g. the "Tuberculist" website sponsored by the Pasteur Institute. In one embodiment of the invention, the antigens from which a selection may be made include but are not limited to all ORF products of *M. tuberculosis* H37Rv as identified in GenBank Accession #AL123456 (NC 000962).

In a preferred embodiment of the invention, the antigens that are expressed by the rBCG are selected from but are not limited to an initial group of 189 antigens presented in Table 1.

**Table 1. List of 189 selected antigens**

| No. | Gene (name) | Size | NCBI Annotation [Updated annotation] ^{(a)} |
|---|---|---|---|
| Rv0079 | | 273 | hypothetical protein |
| Rv0101 | *nrp* | 2512 | probable peptide synthase (nrp) |
| Rv0125 | *pepA* | 355 | probable serine protease pepA (MTB32A) [DegQ] |
| Rv0170 | *mce1B* | 346 | MCE -family protein mce1B [Ttg2C, periplasmic component ABC transporter] |
| Rv0198c | | 663 | possible zinc metalloprotease [PepO, predicted metalloendopeptidase] |
| Rv0211 | *pck4* | 606 | phosphoenolpyruvate carboxykinase pckA (GTP) |
| Rv0227c | | 421 | probable conserved membrane protein |
| Rv0243 | *fadA2* | 440 | acetyl-coA acetyltransferase [beta keto-thiolase] |
| Rv0251c | *hsp* [*acr2*] | 159 | heat-shock protein hsp (heat-stress induced ribosome-binding protein A) |
| Rv0282 | | 631 | hypothetical protein [AAA ATPase] |
| Rv0283 | | 538 | possible conserved membrane protein [ATP/GTP-binding protein] |
| Rv0284 | [*ftsk*] | 1330 | possible conserved membrane protein [chromosome partition ATPase] |
| Rv0285 | *PE5* | 102 | PE family protein (PE5) |
| Rv0286 | *PPE4* | 513 | PPE family protein (PPE4) |
| Rv0287 | *exsG* | 97 | ESAT-6-like protein esxG (conserved hypothetical protein TB9.8) |
| Rv0288 | *esxH* (*TB10.4*) | 96 | low MW protein antigen 7 esxH (10 kDa antigen) CFP-7, TB10.4) |
| Rv0289 | | 295 | hypothetical protein [transporter] |
| Rv0290 | | 472 | probable conserved trans-membrane protein (mgcP3) [transporter] |
| Rv0292 | | 331 | probable conserved trans-membrane protein |
| Rv0350 | *dnaK* | 625 | molecular chaperone DnaK |
| Rv0351 | *grpE* | 235 | probable grpE protein (HSP-70 cofactor) |
| Rv0383c | | 284 | possible conserved secreted protein |
| Rv0384c | *clpB* | 848 | probable endopeptidase ATP binding protein ClpB (chain B) heat-shock protein F84.1 |
| Rv0450c | *mmpl4* | 967 | probable conserved trans-membrane mmpL4 [drug exporting] |
| Rv0467 | *icl* [*aceA*] | 428 | isocitrate lyase (icl) |
| Rv0468 | *fadB2* | 268 | 3-hydroxybutyryl-CoA dehydrogenase |
| Rv0503c | *cmaA2* | 302 | cyclopropane-fatty-acyl-phospholipid-synthase 2 (cyclopropane mycolic acid synthase 2, CMAA2) |
| Rv0569 | | 88 | hypothetical protein |
| Rv0572c | | 113 | hypothetical protein |
| Rv0574c | [*pgsA*] | 380 | hypothetical protein [pgsA poly-gamma-glytamyl biosyntehsis] |
| Rv0588 | *yrbE2B* | 295 | conserved hypothetical integral membrane protein YrbE2B [putative Ttg2B, ABC-type transport system involved in resistance to organic solvents, permease component) |
| Rv0628c | | 383 | hypothetical protein |
| Rv0685 | *Tuf* | 396 | elongation factor Tu, tuf [iron-regulated] |
| Rv0754 | *PE_PGRS11* | 584 | PE-PGRS family protein (PE-PGRS 11) [phosphoglycerate mutase] |
| Rv0798c | *cap29* | 265 | 29 kDa antigen CFP-29 [linocin M-18 bacteriocin] |
| Rv0824c | *desA1* | 389 | probable acyl-[-acyl-carrier-desaturase desA1] |
| Rv0847 | *lpqS* | 130 | probable lipoprotein LPQS |
| Rv0867c | *rpfA* | 407 | possible conserved trans-membrane protein [transglycosylase, rpfA] |
| Rv0885 | | 340 | hypothetical protein |
| Rv1006 | | 567 | hypothetical protein |
| Rv1009 | *rpfB* | 362 | possible resuscitation-promoting factor rpfB [transglycosylase, C5 adhesion domain] |
| Rv1057 | | 393 | hypothetical protein |
| Rv1094 | *desA2* | 271 | possible acyl-[-acyl-carrier protein] desaturase (DESA2) |
| Rv1124 | *ephC* | 316 | probable epoxide hydrolase EPHC (epoxide hydratase) |
| Rv1130 | *[prpD]* | 526 | hypothetical protein [2 methyl-citrate dehydratase] |
| Rv1131 | *gltA1* | 393 | citrate synthase (glaA1) |
| Rv1169c | *PE11* | 100 | PE family protein (PE11) [triacyl glycerol lipase] |
| Rv1174c | [*sak5*] | 110 | low MW T-cell antigen TB8.4 [secretion antigen SA5K] |
| Rv1182 | *papA3* | 472 | probable conserved polyketide synthase associated protein PAPA3 |
| Rv1186c | | 538 | hypothetical protein [regulator of polyketide synthase expression] |
| Rv1187 | *rocA* | 543 | probable proline-5-carboxylate dehydrogenase rocA |
| Rv1188 | | 329 | probable proline dehydrogenase |
| Rv1196 | *PPE18* (*mtb39a*) | 391 | probable proline dehydrogenase PPE family protein [MTB39a] |
| Rv1221 | *sigE* | 257 | RNA polymerase sigma-70 factor (SigE) |
| Rv1347c | | 210 | hypothetical protein [GCN5-related N-acetyltransferase fold] |
| Rv1348 | [*IrtA*] | 859 | probable drug-transport trans-membrane ATP-binding protein ABC-transporter [MdlA/MsbA essential ABC transporter, siderophore interaction protein] |
| Rv1349 | [*irtB*] | 579 | possible drug-transport trans-membrane ATP-binding protein ABC-transporter [ATM1 ABC siderophore-iron transporter] |
| Rv1411c | *lprG* | 236 | possible conserved lipoprotein lprG |
| Rv1436 | *gap* | 339 | glyceraldehyde-3-phosphate dehydrogenase |
| Rv1461 | [*sufB*] | 846 | hypothetical protein [sufB, cytosine desulfurase activator] |
| Rv1462 | [*sufD*] | 397 | hypothetical protein [sufD, cytosine desulfurase activator] |
| Rv1464 | *csd* [*sufS*] | 417 | possible cysteine desulfurase csd [SufS] |
| Rv1465 | [*nifU*] | 162 | possible nitrogen fixation related protein [IscU] |
| Rv1466 | | 115 | hypothetical protein [PaaD, predicted metal-sulfur cluster biosynthetic enzyme] |
| Rv1477 | *ripA* | 427 | hypothetical invasion protein [Nlp_p60 cell-wall hydrolase] |
| Rv1478 | | 241 | hypothetical invasion protein [NLp_p60 cell-wall hydrolase] |
| Rv1594 | *nadA* | 349 | quinoline synthetase (nadA) |
| Rv1636 | *TB15.3* | 146 | iron-regulated conserved hypothetical protein TB15.3 [USP] |
| Rv1733c | | 210 | probable conserved hansmembrane protein |
| Rv1734c | | 80 | hypothetical protein [dihydrolysine acetyl-transferase] |
| Rv1735c | | 165 | hypothetical membrane protein |
| Rv1736c | *narX* | 652 | possible nitrate reductase narX |
| Rv1737c | *narK2* | 395 | possible nitrate/nitrite transporter narK2 |
| Rv1738 | | 94 | hypothetical protein |
| Rv1793 | *esxN* | 94 | putative ESAT-6-like protein ESXN (ESAT-6-like protein 5) |
| Rv1812c | [*ndH*] | 400 | possible dehydrogenase [Ndh, NADH dehydrogenase, FAD-containing subunit] |
| Rv1813c | | 143 | hypothetical protein |
| Rv1876 | *bfrA* | 159 | probable bacterioferritin bfrA |
| Rv1884c | *rpfC* | 176 | probable resuscitation-promoting factor rpfC [transglycosylase] |
| Rv1886c | *ftpB* (*Ag85B*) | 325 | secreted antigen 85-B FBPB (85-B) (mycolyl-transferase 85B) |
| Rv1908c | *katG* | 740 | catalase-peroxidase-peroxinitritase-T katG |
| Rv1926c | *mpt63* | 159 | immunogenic protein MPT63 (16 kDa immunoprotective extracellular antigen) |
| Rv1980c | *mpb64* | 228 | immunogenic protein MPT64 |
| Rv1986 | | 199 | probable conserved integral membrane protein [lysine efflux permease] |
| Rv1996 | | 317 | hypothetical protein [USP] |
| Rv1997 | *ctpF* | 905 | probable metal cation transporter P-type APTase cptF |
| Rv1998c | | 258 | hypothetical protein |
| Rv2004c | | 498 | hypothetical protein [predicted kinase] |
| Rv2005c | | 295 | hypothetical protein [USP-like] |
| Rv2006 | *otsB1* | 1327 | probable trehalose-6-phosphate phosphatase OSTB1 |
| Rv2007c | *fdxA* | 114 | probable ferrodoxin fdxA |
| Rv2008c | | 441 | hypothetical protein [predicted ATPase] |
| Rv2011c | | 143 | hypothetical protein[transcription regulator] |
| Rv2028c | | 279 | hypothetical protein [USP] |
| Rv2029c | *pfkB* | 339 | possible phosphofructokinase (pfkB) |
| Rv2030c | | 681 | hypothetical protein [putative esterase/transferase] |
| Rv2031c | *acr* (α-crystallin) | 144 | heat-shock protein HspX (alpha-crystallin homolog) 14kDa antigen Hsp16.3 |
| Rv2032 | *acg* | 331 | conserved hypothetical protein Acg |
| Rv2110c | [*prcB*] | 291 | proteosome (beta subunit) PrcB [HslV protease] |
| Rv2123 | *PPE37* | 473 | PPE family protein (PPE37) |
| Rv2140c | *TB18.6* [*pepB*] | 176 | hypothetical protein (TB 18.6) [PEBP, bacterial/archeal phosphatidylethanolamine-binding protein] |
| Rv2182c | [*pslC*] | 247 | 1-acylglycerol-3-phosphate O-acyltransferase |
| Rv2224c | [*caeA*] | 520 | probable exported protease [cae, carboxylase A, TAP-like protein] |
| Rv2244 | *acpM* | 115 | acyl-carrier protein (acpM) |
| Rv2245 | *kasA* | 416 | 3-oxoacyl-(acyl carrier protein) synthase (kasA) |
| Rv2246 | *kasB* | 438 | 3-oxoacyl-(acyl carrier protein) synthase (kasB) |
| Rv2251 | [*glcD*] | 475 | possible flavoprotein [GlcD, FAD/FMN-containing dehydrogenases] |
| Rv2377c | *mbtH* | 71 | putative conserved protein MBTH |
| Rv2378c | *mbtG* | 431 | lysine-N-oxygenase MBTG [lucD] |
| Rv2380c | *mbtE* | 1682 | peptide synthase MBTE [EntF] |
| Rv2381c | *mbtD* | 1004 | polyketide synthase MBTD [acyl-transferase, KR domain] |
| Rv2382c | *mbtC* | 444 | polyketide synthase MBTC |
| Rv2383c | *mbtB* | 1414 | phenyloxazoline synthase (MBTB) [EntF, GrsT] |
| Rv2386c | *mbtI* | 450 | anthranilate synthase component I (MBTA) [salicylate synthase] |
| Rv2389c | *rpfD* | 154 | probable resuscitation-promoting factor rpfE [transglycosylase] |
| Rv2428 | *ahpC* | 195 | alkyl hydroxyperoxide reductase C protein ahpC |
| Rv2429 | *ahpD* | 177 | alkyl hydroxyperoxide reductase D protein ahpD |
| Rv2430c | *PPE41* | 194 | PPE family protein (PPE41) |
| Rv2450c | *rpfE* | 172 | probable resuscitation-promoting factor rpfE [transglycosylase] |
| Rv2457c | *clpX* | 426 | ATP-dependent protease ATP-binding subunit (CplX) |
| Rv2466c | | 207 | hypothetical protein [putative DsbA_FmE] [thiol oxidoreductase, polyketide biosynthesis] |
| Rv2510c | | 533 | hypothetical protein [ATP binding domain] |
| Rv2515c | | 415 | hypothetical protein [putative zinc peptidase] |
| Rv2516c | | 250 | hypothetical protein |
| Rv2557 | | 224 | hypothetical protein |
| Rv2590 | *fadD9* | 1168 | probable fatty-acid coA ligase fadD9 |
| Rv2620c | | 141 | probable conserved trans-membrane protein |
| Rv2621c | | 224 | possible transcriptional regulatory protein |
| Rv2622 | | 273 | possible methyltransferase (methylase) |
| Rv2623 | *TB31.7* | 297 | hypothetical protein TB31.7 [USP] |
| Rv2625c | | 393 | probable conserved trans-membrane alanine-rich and leucine-rich protein [zinc-protease M-50 CBS domain] |
| Rv2626c | | 143 | hypothetical protein [CBS pair-binding/regul, euk] |
| Rv2627c | | 413 | hypothetical protein |
| Rv2628 | | 120 | hypothetical protein |
| Rv2629 | | 374 | hypothetical protein [peptide chain release factor erF1] |
| Rv2657c | | 86 | probable phiRv2 prophage protein [MerR regulatory protein] |
| Rv2659c | | 375 | probable phiRv2 prophage integrase |
| Rv2660 | | 75 | hypothetical protein |
| Rv2710 | *sigB* | 323 | RNA polymerase sigma factor (SigB) |
| Rv2744c | *35kd-Ag* [*pspA*] | 270 | conserved 35 kDa alanine-rich protein [phage-shock protein IM30] |
| Rv2780 | *ald* | 371 | secreted L-alanine dehydrogenase ald (40kDa antigen, TB43) |
| Rv2833c | *ugpB* | 436 | probable Sn-glycerol-3-phosphate-binding lipoprotein UGPB |
| Rv2856 | *nicT* | 372 | possible nickel-transport integral membrane protein nicT |
| Rv2869c | | 404 | probable conserved trans-membrane protein [putative pdz membrane associated zinc-metalloprotease] |
| Rv2875 | *mpt70* | 193 | major secreted immunogenic protein MPT70 |
| Rv2930 | *fadD26* | 626 | fatty-acid-coA ligase FadD26 |
| Rv2986c | *hupB* | 214 | probable DNA-binding protein HU homolog HupB (histone-like protein, 21kDa laminin-2 binding protein) |
| Rv2999 | *lppY* | 321 | probable conserved lipoprotein LPPY |
| Rv3126c | | 104 | hypothetical protein |
| Rv3127 | | 344 | hypothetical protein [possible nitroreductase] |
| Rv3129 | | 110 | hypothetical protein |
| Rv3130c | *tgsI* | 463 | hypothetical protein [diacylglycerol acyltransferase] |
| Rv3131 | *nfnB* | 332 | hypothetical protein [possible nitroreductase NfnB] |
| Rv3132c | *devS* | 578 | two-component sensor histidine kinase DevS |
| Rv3133c | *devR* | 217 | two-component transcription regulatory protein DevR |
| Rv3134c | | 268 | hypothetical protein [USP] |
| Rv3139 | *fadE24* | 468 | probable acyl-coA dehydrogenase FadE24 |
| Rv3140 | *fadE23* | 401 | probable acyl-coA dehydrogenase FadE23 |
| Rv3173c | | 200 | probable transcriptional regulatory protein (TetR/acRR family) |
| Rv3229c | *desA3* | 427 | possible linoleoyl-coa desaturase (delta-(6)-desaturase) |
| Rv3250c | *rubB* | 495 | probable rubredoxin rubB |
| Rv3251c | *rubA* | 55 | probable rubredoxin rubA |
| Rv3283 | *sseA* | 297 | probable thiosulfate sulfurtranserase SSEA (rhodanase) |
| Rv3290c | *lat* | 449 | L-lysine epsilon aminotransferase |
| Rv3347c | *PPE55* | 3157 | PPE family protein (PE55) [8 copies pentapeptide repeats] |
| Rv3372 | *ostB2* | 391 | possible trehalose-6-phosphate phosphatase (OSTB2) |
| Rv3515c | *fadD19* | 548 | AMP-dependent fatty-acid-coA ligase FadD19 |
| Rv3516 | *echA19* | 263 | enoyl-coA hydratase/isomerase (echA19) |
| Rv3546 | *fadA5* | 391 | acetyl-coA acetyltransferase (FadA5) |
| Rv3570c | [*ncnH*] | 394 | possible oxidoreductase [NcnH, naphthocyclinone hydroxylase] |
| Rv3593 | *lpqF* [*penP*] | 452 | probable conserved lipoprotein LPQF [PenP, beta-lactamase class A] |
| Rv3597c | *lsr2* | 112 | probable iron-regulated LSR2 protein precursor |
| Rv3616c | [*espB*] | 392 | conserved hypothetical alanine-rich and glycine-rich protein [ESAT secretion system component] |
| Rv3619c | *esxV* | 94 | putative ESAT-6-like protein ESXV (ESAT-6-like protein 1) |
| Rv3660c | | 350 | hypothetical protein |
| Rv3763 | *lpqH* | 159 | 19 kDa lipoprotein antigen precursor LPQH |
| Rv3804c | *fbpA* (*Ag85A*) | 338 | secreted antigen 85-A FBPA (85-A) (mycolyl-transferase 85A) |
| Rv3812 | *PE_PGRS62* | 504 | PE-PGRS family protein (PE_PGRS62) |
| Rv3833 | | 263 | transcriptional regulatory protein [probable araC family] |
| Rv3839 | | 258 | hypothetical protein |
| Rv3840 | | 137 | probable transcriptional regulatory protein [cell-envelope related transcription attenuator] |
| Rv3841 | *bfrB* | 181 | possible bacterioferritin bfrB |
| Rv3871 | [*Ftsk*] | 591 | hypothetical protein [FtsK_SPOIIIE] |
| Rv3873 | *PPE68* | 368 | PPE family protein [PPE68, RD1 T/B immunogen] |
| Rv3874 | *esxB* | 100 | 10 kDa culture filtrate antigen ESXB (LHP, CFP-10) |
| Rv3875 | *esxA* | 95 | 6 kDa early secretory antigenic target ESXA (ESAT-6) |
| Rv3876 | [*Ftsk*] | 666 | conserved hypothetical proline and alanine-rich protein [chromosome partitioning ATPase] |
| Rv3878 | | 280 | conserved hypothetical alanine-rich protein |
| Rv3879c | | 729 | hypothetical alanine and proline-rich protein |

| | | | |
|---|---|---|---|
| (a). NCBI annotation is based on Accession # AL123456 (NC_000962); updated annotation is based on bioinformatic analyses, data from MTB-related servers and experimental evidence. | | | |

In a more preferred embodiment of the invention, the antigens that are expressed by the rBCG are the 45 antigens presented in Tables 2 and 3.

**Table 2. Top-ranking antigens (according to Class/Phase)**

| No. | Gene (name) | Size | NCBI annotation [Updated annotation]^{(a)} | Score Qual | Score Quant |
|---|---|---|---|---|---|
| Class: DORMANCY/DosR | | | | | |
| Rv1738 | | 94 | hypothetical protein | 9 | 14 |
| Rv2623 | *TB31.7* | 297 | heat-shock protein TB31.7 [universal stress protein] | 9 | 14 |
| Rv2031c | *acr* (α-crystalllin) | 144 | heat-shock protein HspX (alpha-crystallin homolog) 14kDa antigen Hsp16.3 | 8 | 14 |
| Rv2032 | *acg* | 331 | conserved hypothetical protein Acg [nitroreductase] | 8 | 13 |
| Rv2626c | | 143 | hypothetical protein [CBS pair-binding/regulation, euk] | 8 | 13 |
| Rv2005c | | 295 | hypothetical protein [USP-like] | 8 | 12 |
| Rv3127 | | 344 | hypothetical protein [possible nitroreductase] | 8 | 12 |
| Rv1733c | | 210 | probable conserved trans-membrane protein | 8 | 11 |
| Rv1996 | | 317 | hypothetical protein [USP] | 8 | 10 |
| Rv2628 | | 120 | hypothetical protein | 8 | 9 |
| Rv0079 | | 273 | hypothetical protein | 7 | 11 |
| Rv3130c | [*tgsI*] | 463 | hypothetical protein [diacylglycerol acyltransferase] | 7 | 11 |
| Rv3131 | [n*fnB*] | 332 | hypothetical protein [possible nitroreductase NfnB] | 7 | 11 |
| Rv1813c | | 143 | hypothetical protein | 7 | 9 |
| Rv2006 | *otsB1* | 1327 | probable trehalose-6-phosphate phosphatase OTSB1 | 7 | 9 |
| Rv2029c | *pfkB* | 339 | possible phophofructokinase (pfkB) | 7 | 9 |
| Rv2627c | | 413 | hypothetical protein [serine endopeptidase] | 7 | 9 |
| Rv2030c | | 681 | hypothetical protein [putative esterase/transferase] | 6 | 10 |
| Rv3132c | *devs* | 578 | two component sensor histidine kinase DEVS | 6 | 10 |
| Rv2629 | | 374 | hypothetical protein [peptide release factor erf-1] | 6 | 9 |

| Class: RESUSCITATION | | | | | |
|---|---|---|---|---|---|
| Rv2450c | *rpfE* | 172 | probable resuscitation-promoting factor rpfE [transglycosylase] | 9 | 14 |
| Rv1009 | *rpfB* | 362 | possible resuscitation-promoting factor rpfB [transglycosylase, C5 adhesion domain] | 9 | 13 |
| Rv0867c | *rpfA* | 407 | possible conserved trans-membrane protein [transglycosylase, rpfA] | 9 | 12 |
| Rv2389c | *rpfD* | 154 | probable resuscitation-promoting factor rpfD [transglycosylase] | 8 | 10 |
| Rv1884c | *rpfC* | 176 | probable resuscitation-promoting factor rpfC [transglycosylase] | 7 | 8 |

| Class: REACTIVATION | | | | | |
|---|---|---|---|---|---|
| Rv1009 | *rpfB* | 362 | possible resuscitation-promoting factor rpfB [transglycosylase, C5 adhesion domain] | 9 | 13 |
| Rv0867c | *rpfA* | 407 | possible conserved trans-membrane protein [transglycosylase, rpfA] | 9 | 12 |
| Rv0288 | *esxH (TB10.4)* | 96 | low MW protein antigen 7 esxH (10kDa antigen) CFP-7, TB10.4) | 8 | 13 |
| Rv0685 | *Tuf* | 396 | elongation factor Tu [iron-regulated] | 8 | 9 |
| Rv0824c | *desAI* | 389 | probable acyl[-acyl-carrier-desaturase desa1] | 7 | 10 |
| Rv2744c | *35kd-Ag* [*pspA*] | 270 | conserved 35 kDa alanine-rich protein [phage-shock protein IM30] | 7 | 8 |
| Rv3347c | *PPE55* | 3157 | PPE 55 Family Protein [8 copies pentapeptide repeats] | 6 | 10 |
| Rv1130 | *prpD* | 526 | hypothetical protein [2 methyl-citrate dehydratase] | 6 | 9 |
| Rv1169c | *PE11* | 100 | PE family protein (PE11) [triacyl glycerol lipase] | 6 | 9 |

| Class: CLASSICAL | | | | | |
|---|---|---|---|---|---|
| Rv1886c | *fbpB (Ag85B)* | 325 | mycolyl transferase/fibronectin binding | 8 | 14 |
| Rv1980c | *mpb64* | 228 | antigen MPT64/MPB64 | 7 | 13 |
| Rv3804c | *fbpA (Ag85A)* | 338 | mycolyl transferase/fibronectin binding | 7 | 13 |
| Rv3875 | *esxA* | 95 | 6kDa early secretory antigen esxA | 6 | 11 |
| Rv1926c | *mpt63* | 159 | immunogenic protein MPT63 (16 kDa immunoprotective extracellular protein) | 6 | 10 |
| Rv0467 | *icl* | 428 | isocitrate lyase (icl) [AceA] | 6 | 9 |

| Class: OTHERS | | | | | |
|---|---|---|---|---|---|
| Rv3873 | *PPE68* | 368 | hypothetical protein | 8 | 13 |
| Rv1908c | *katG* | 740 | catalase-peroxidase-peroxinitritase-T (KATG heme dependent) | 7 | 10 |
| Rv1174c | *sak5* | 110 | low MW T-cell antigen TB8.4 (secreted) | 7 | 9 |
| Rv1349 | *irtB* | 579 | probable drug transport ATP-binding protein ABC transporter [ATM1 ABC siderophor-iron transporter] | 7 | 9 |
| Rv2780 | *ald* | 371 | secreted L-alanine dehydrogenase ALD (40kDa antigen) (TB4.3, cell associated pyridine nucleotide transhydrogenase) | 7 | 9 |
| Rv2620c | | 141 | probable conserved transmembrane protein | 7 | 8 |
| Rv1793 | *esxN* | 94 | putative ESAT-6 like protein (ESXN, ESAT-6 like protein 5) | 6 | 9 |

| | | | | | |
|---|---|---|---|---|---|
| (a). NCBI annotation is based on Accession # AL123456 (NC_000962); updated annotation is based on bioinformatic analyses, data from MTB-related servers and experimental evidence. | | | | | |

The list of 45 high-ranking antigens were classified according to the following classes:
* DosR regulon genes (Voskuil et al., 2003, J. Exp Med 198(5):705-713; Voskuil et al. 2004, Tuberculosis 84, 218-227)
* Resuscitation genes
* Reactivation genes (essentially according to Talaat et al., 2007, Proc. Natl. Acad. Sci. 189(21):7877-7886)
* Classical genes
* Others (mainly involved in persistence and stress response)

Some of the antigens appear in more than one class in Table 3.

**Table 3. Top-ranking antigens sorted by qualitative and quantitative scores**

| No. | Gene (Name) | Size | NCBI Annotation [Updated annotation]^{(a)} | Score (Qual) | Score (Quant) | Class/Phase |
|---|---|---|---|---|---|---|
| GROUP 1 | | | | | | |
| Rv1738 | | 94 | hypothetical protein | 9 | 14 | DosR |
| Rv2450c | *rpfE* | 172 | probable resuscitation-promoting factor rpfE [transglycosylase] | 9 | 14 | Resuscitation |
| Rv2623 | *TB31.7* | 297 | hypothetical protein TB31.7 [USP] | 9 | 14 | DosR |
| Rv1009 | *rpfB* | 362 | possible resuscitation-promoting factor rpfB [transglycosylase, C5 adhesion domain] | 9 | 13 | Reactivation Resuscitation |
| Rv0867c | *rpfA* | 407 | possible conserved trans-membrane protein [transglycosylase, rpfA] | 9 | 12 | Reactivation Resuscitation |
| Rv2031c | *acr* (*α-crystallin)* | 144 | heat-shock protein HspX (α-crystallin homolog) | 8 | 14 | DosR |
| | | | 14kDa antigen Hsp 16.3 | | | |
| Rv1886c | *fbpB* (Ag85B) | 325 | secreted antigen 85-B FBPB (85-B) (mycolyl-transferase 85B) | 8 | 14 | Classical |
| Rv0288 | *esxH* (TB10.4) | 96 | low MW protein antigen 7 esxH (10 kDa antigen) CFP-7, TB10.4) | 8 | 13 | Reactivation |
| Rv2032 | *acg* | 331 | conserved hypothetical protein Acg [nitroreductase] | 8 | 13 | DosR |
| Rv2626c | | 143 | hypothetical protein [CBS pair-binding/regulation, euk] | 8 | 13 | DosR |
| Rv3873 | *PPE68* | 368 | PPE family protein [PPE68, RD1 T/B immunogen] | 8 | 13 | Others |
| Rv2005c | | 295 | hypothetical protein [USP-like] | 8 | 12 | DosR |
| Rv3127 | | 344 | hypothetical protein [possible nitroreductase] | 8 | 12 | DosR |

| GROUP II | | | | | | |
|---|---|---|---|---|---|---|
| Rv1733c | | 210 | probable conserved trans-membrane protein | 8 | 11 | DosR |
| Rv1996 | | 317 | hypothetical protein [USP] | 8 | 10 | DosR |
| Rv2389c | *rpfD* | 154 | probable resuscitation-promoting factor rpfD [tramglycosylase] | 8 | 10 | Resuscitation |
| Rv0685 | *Tuf* | 396 | elongation factor Tu [iron-regulated] | 8 | 9 | Reactivation |
| Rv2628 | | 120 | hypothetical protein | 8 | 9 | DosR |
| Rv1980c | *mpb64* | 228 | immunogenic protein MPT64 | 7 | 13 | Classical |
| Rv3804c | *fbpA* (Ag85A) | 338 | secreted antigen 85-A FBPA (85-A) (mycolyl-transferase 85A) | 7 | 13 | Classical |
| Rv0079 | | 273 | hypothetical protein | 7 | 11 | DosR |
| Rv3130c | [*tgsI*] | 463 | hypothetical protein [diacylglycerol acyltransferase] | 7 | 11 | DosR |
| Rv3131 | [*nfnB*] | 332 | hypothetical protein [possible nitroreductase NfnB] | 7 | 11 | DosR |
| Rv0824c | *desA1* | 389 | probable acyl[-acyl-carrier-desaturase desA1] | 7 | 10 | Reactivation |
| Rv1908c | *katG* | 740 | catatase-peroxidase-peroxinitritase-T katG | 7 | 10 | Others |
| Rv1174c | [*sak5*] | 110 | Low Mw T-cell antigen TB8.4 [secretion antigen SA5K] | 7 | 9 | Others |
| Rv1349 | [*irtB*] | 579 | probable drug transport ATP-binding protein ABC transporter [ATM1 ABC siderophore-iron transporter] | 7 | 9 | Others |
| Rv1813c | | 143 | hypothetical protein | 7 | 9 | DosR |
| Rv2006 | *otsB1* | 1327 | probable trehalose-6-phosphate phosphatase OSTB1 | 7 | 9 | DosR |
| Rv2029c | *pfkB* | 339 | possible phosphofructokinase (pfkB) | 7 | 9 | DosR |
| Rv2627c | | 413 | hypothetical protein [serine endopeptidase | 7 | 9 | DosR |
| Rv2780 | *ald* | 371 | secreted L-alanine dehydrogenase ald (40kDa antigen, TB43) | 7 | 9 | Others |

| GROUP III | | | | | | |
|---|---|---|---|---|---|---|
| Rv1884c | *rpfC* | 176 | probable resuscitation-promoting factor rpfC [transglycosylase] | 7 | 8 | Resuscitation |
| Rv2620c | | 141 | probable conserved transmembrane protein | 7 | 8 | Others |
| Rv2744c | *35kd-Ag* [*pspA*] | 270 | conserved 35 kDa alanine-rich protein [phage-shock protein IM30] | 7 | 8 | Reactivation |
| Rv3875 | *esxA* | 95 | 6kDA early secretory antigenic target ESXA (ESAT-6) | 6 | 11 | Classical |
| Rv1926c | *mpt63* | 159 | immunogenic protein MPT63 (16 kDa immunoprotective extracellular protein) | 6 | 10 | Classical |
| Rv2030c | | 681 | hypothetical protein [putative esterase/transferase] | 6 | 10 | DosR |
| Rv3132c | *devs* | 578 | two component sensor histidine kinase DEVS | 6 | 10 | DosR |
| Rv3347c | *PPE55* | 3157 | PPE family protein (PE55) [8 copies pentapeptide repeats] | 6 | 10 | Reactivation |
| Rv0467 | *icl* | 428 | isocitrate lyase(icl) [AceA] | 6 | 9 | Classical |
| Rv1130 | [*prpD*] | 526 | hypothetical protein [2 methyl-citrate dehydratase] | 6 | 9 | Reactivation |
| Rv1169c | *PE11* | 100 | PE family protein (PE11) [triacyl glycerol lipase] | 6 | 9 | Reactivation |
| Rv1793 | *esxN* | 94 | putative ESAT-6-like protein ESXN (ESAT-6-like protein 5) | 6 | 9 | Others |
| Rv2629 | | 374 | hypothetical protein [peptide release factor erF1] | 6 | 9 | DosR |

| | | | | | | |
|---|---|---|---|---|---|---|
| (a). NCBI annotation is based on Accession # AL123456 (NC_000962); updated annotation is based on bioinformatic analyses, data from MTB-related servers and experimental evidence. | | | | | | |

The list of 45 high-ranking antigens were sorted by the qualitative score and then by the quantitative score according to this invention and as discussed in Example 1. This method leads to 3 groups as follows:
Group I: antigens with qualitative scores 9-8, and within the antigens with a qualitative score of 8, the quantitative score cutoff is 12.
Group II: antigens with qualitative scores 8-7, the quantitative score cutoff of 7 is 9.
Group III: antigens with qualitative score 7-6, the quantitative score cutoff of 6 is 9. Antigens with more than 5 transmembrane segments were removed from the list.

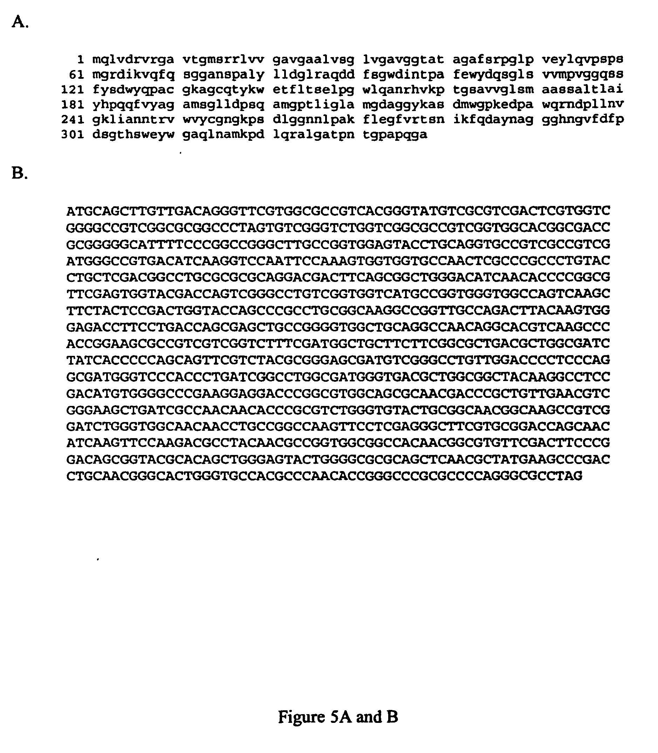

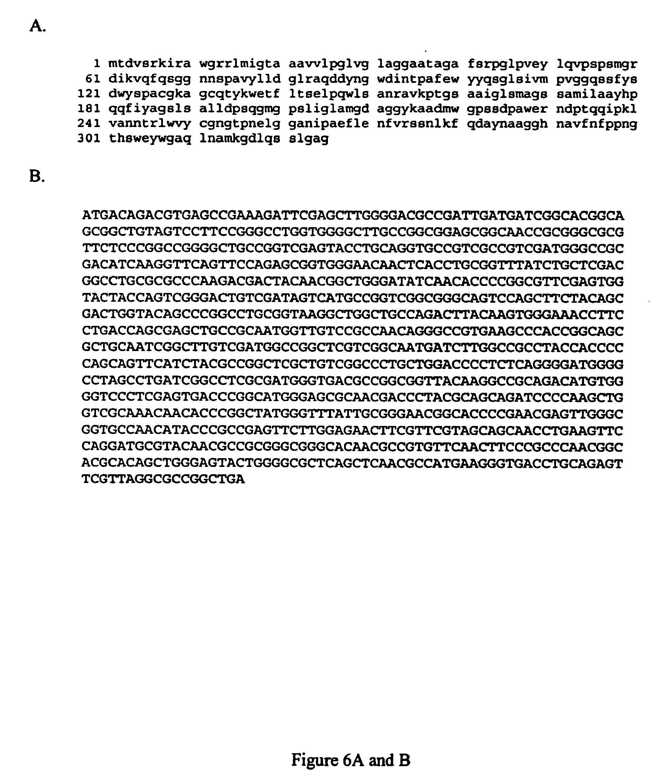

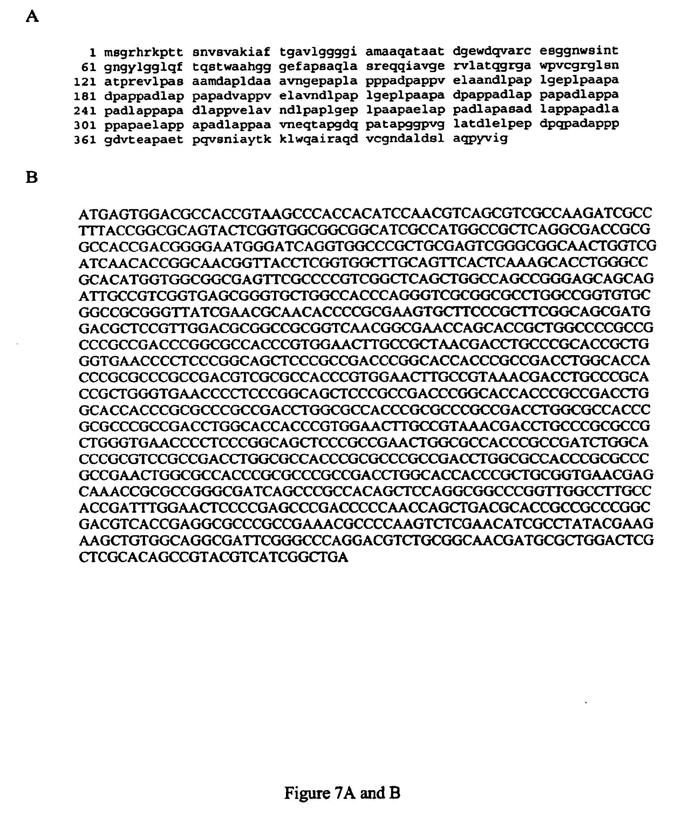

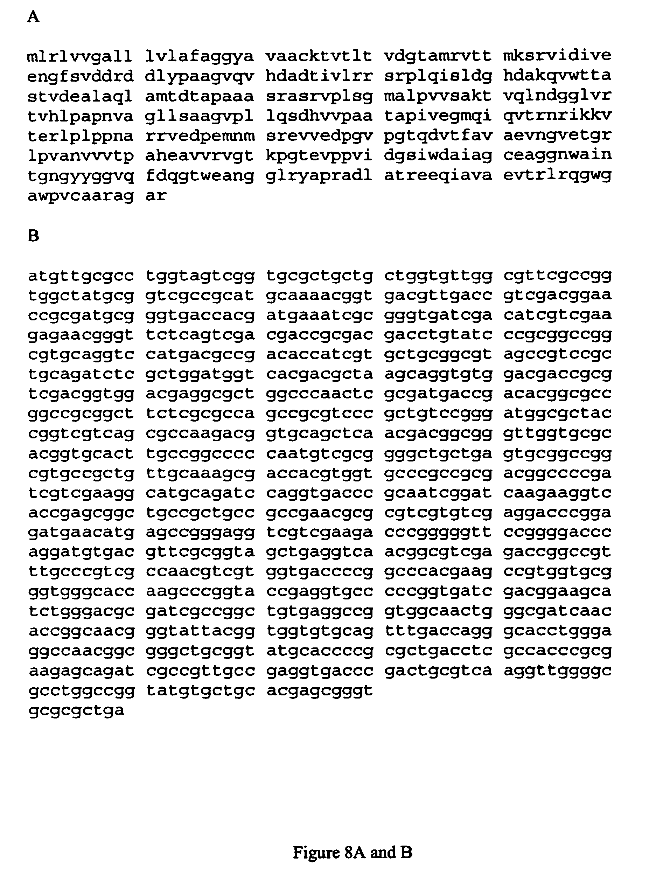

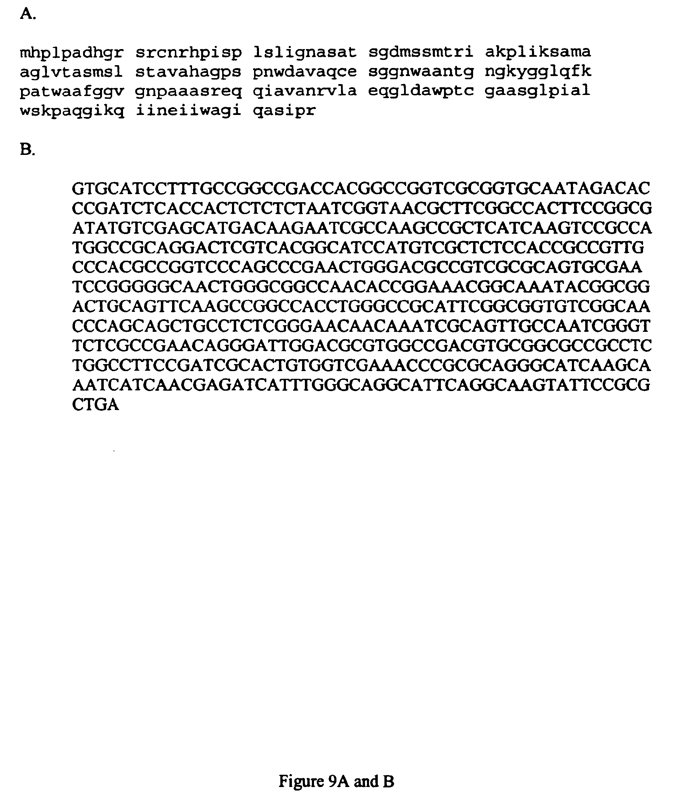

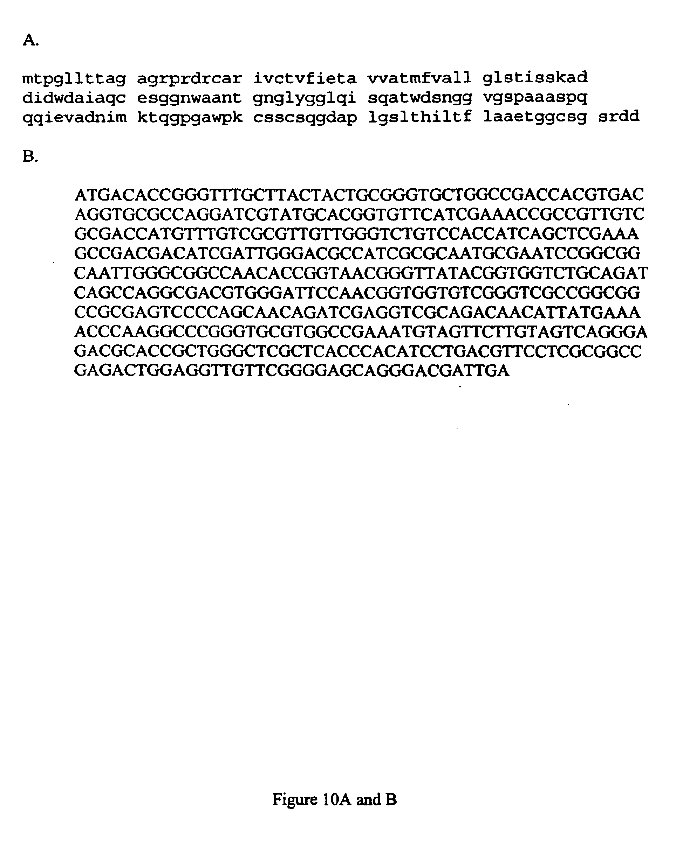

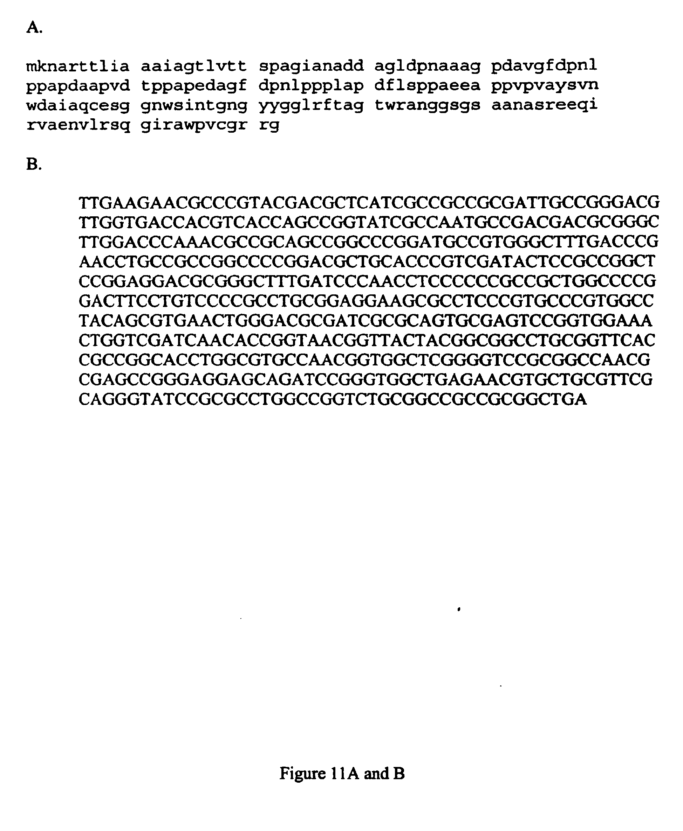

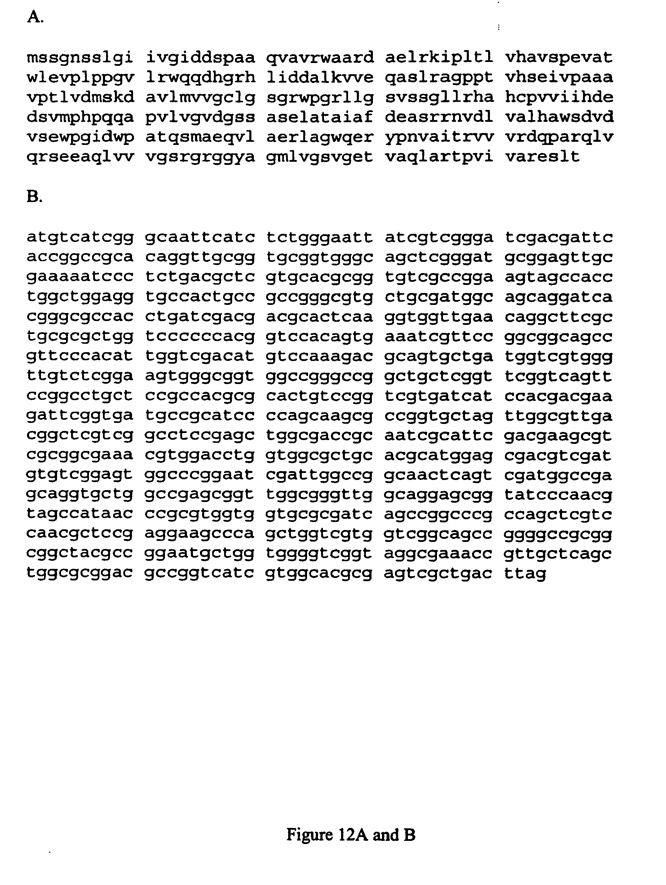

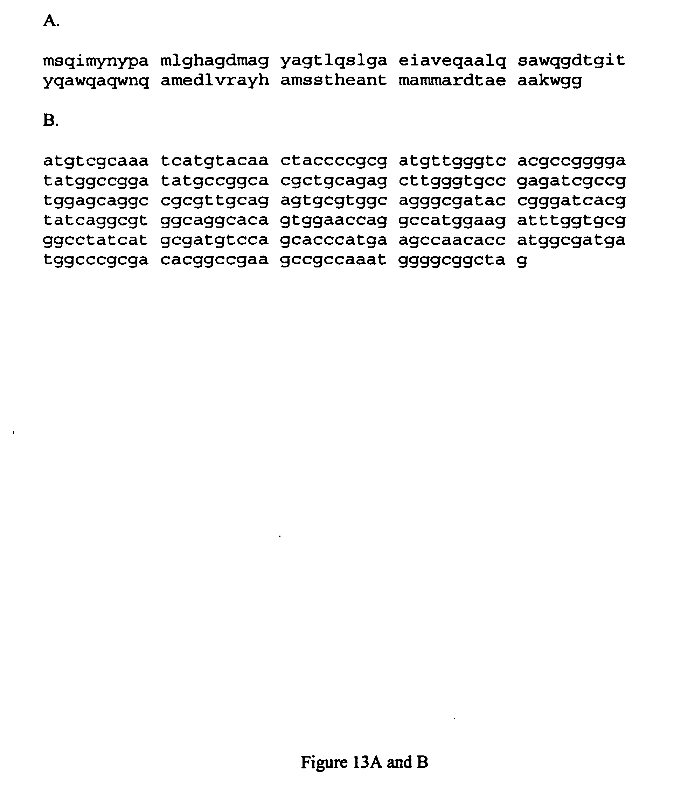

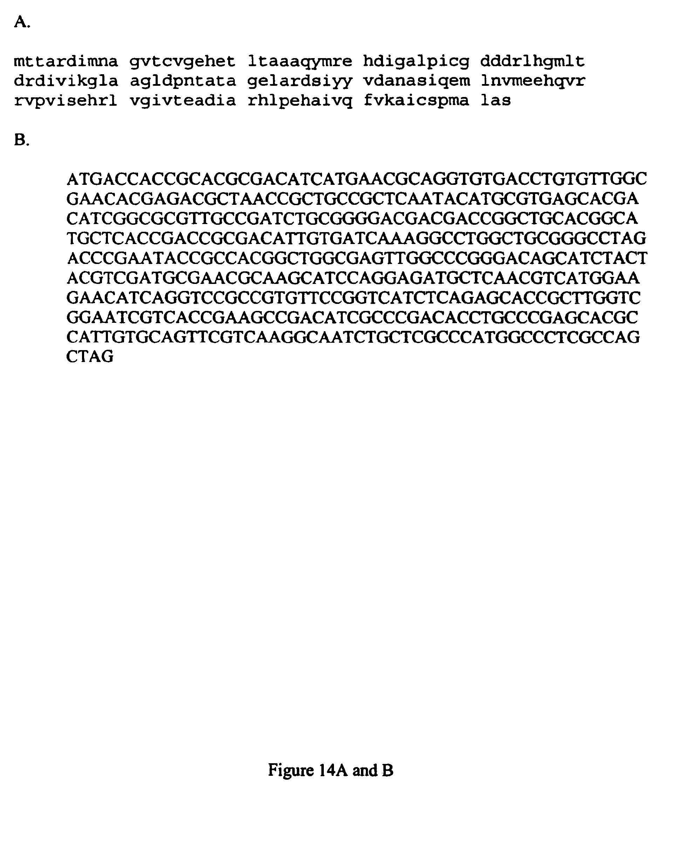

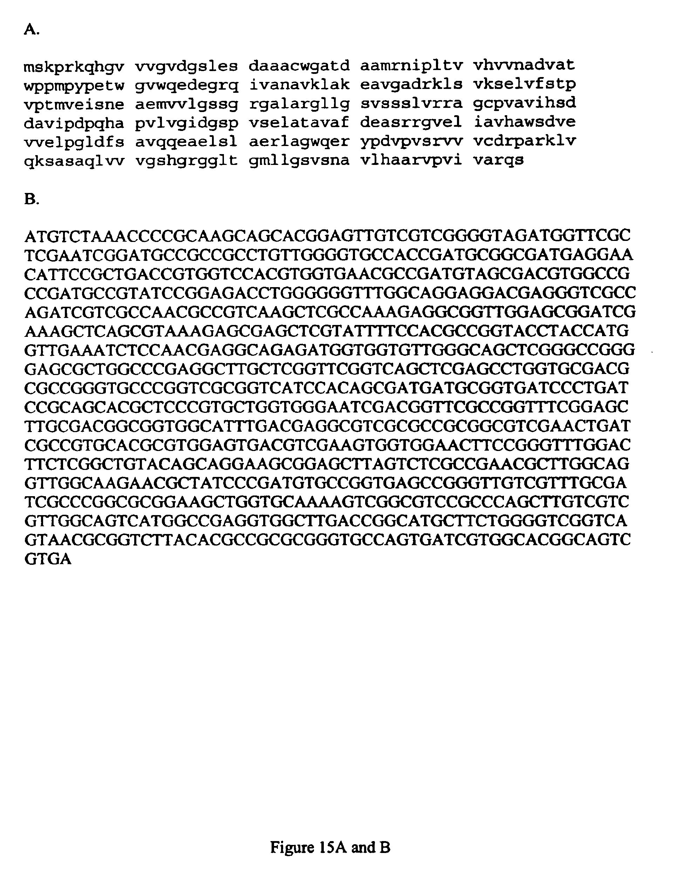

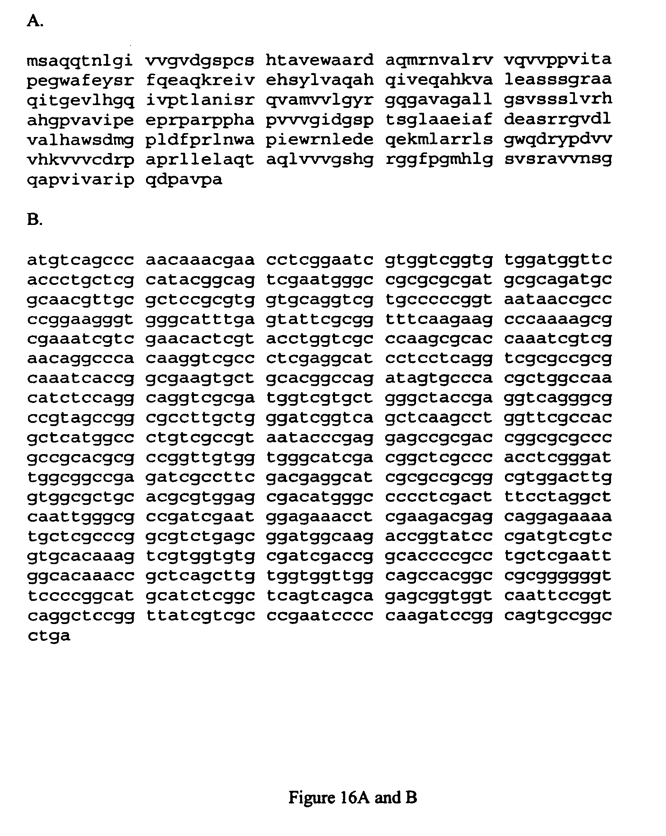

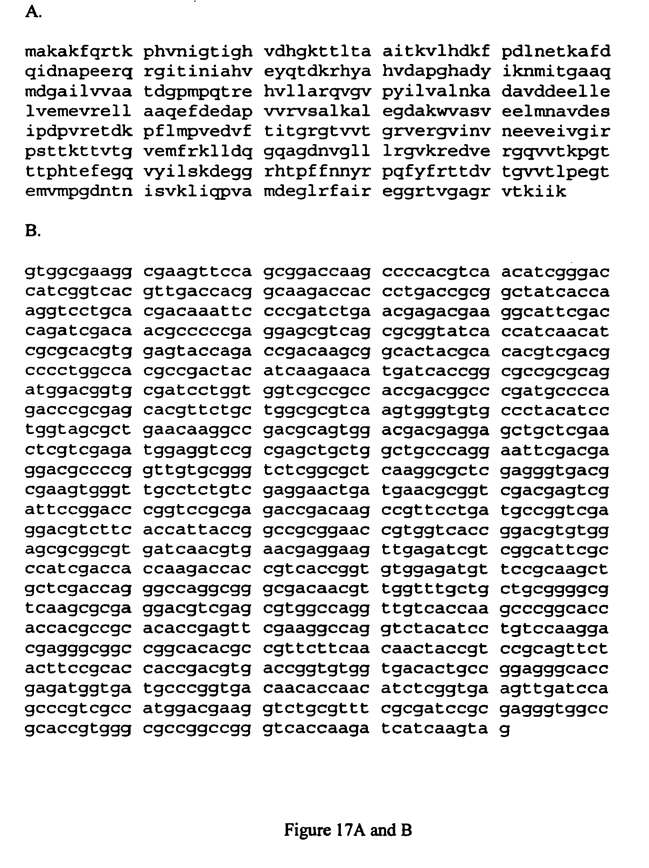

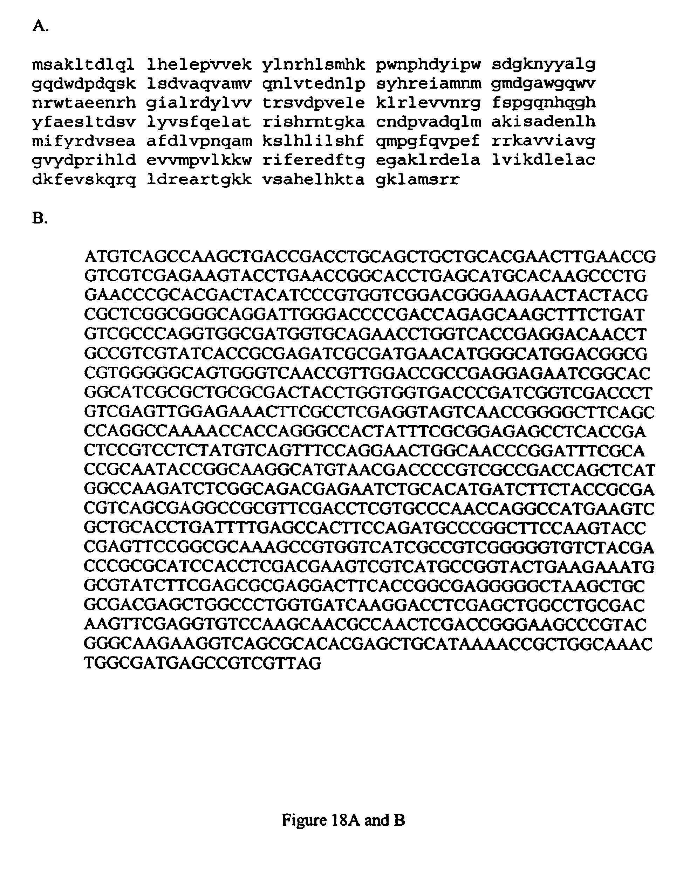

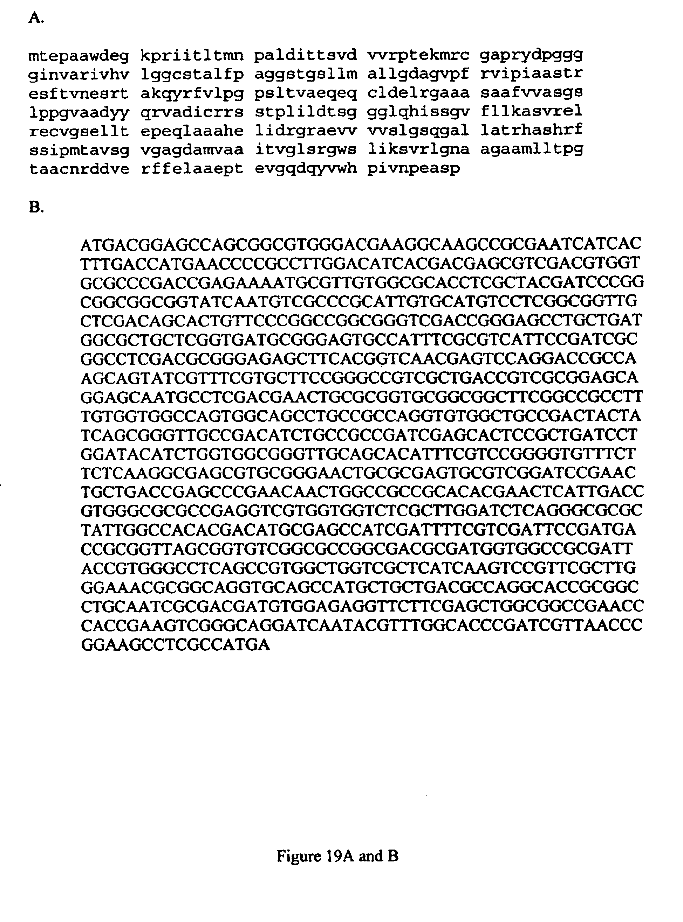

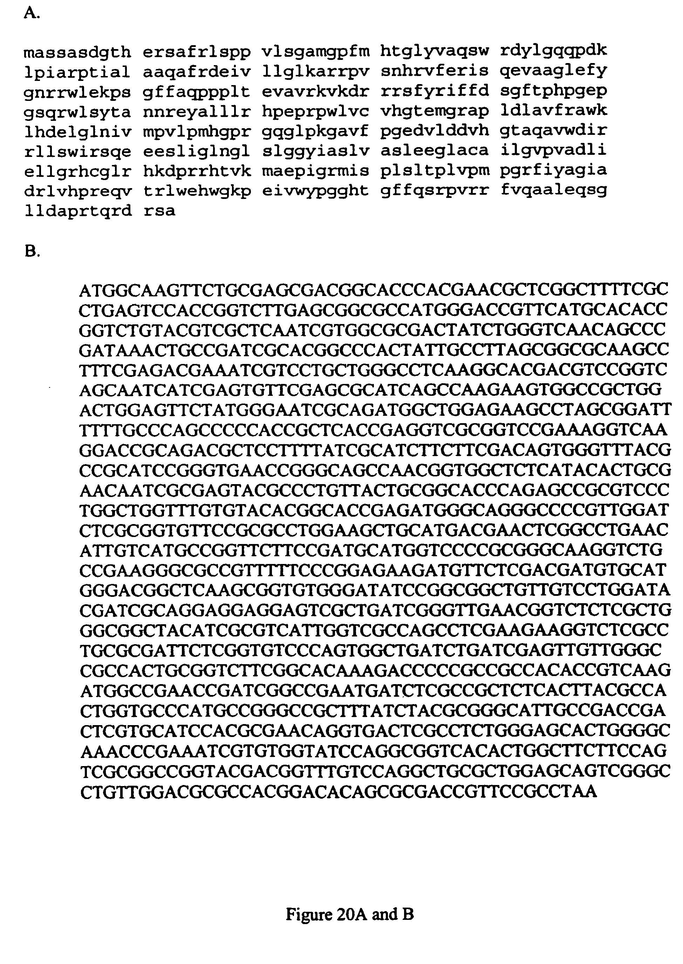

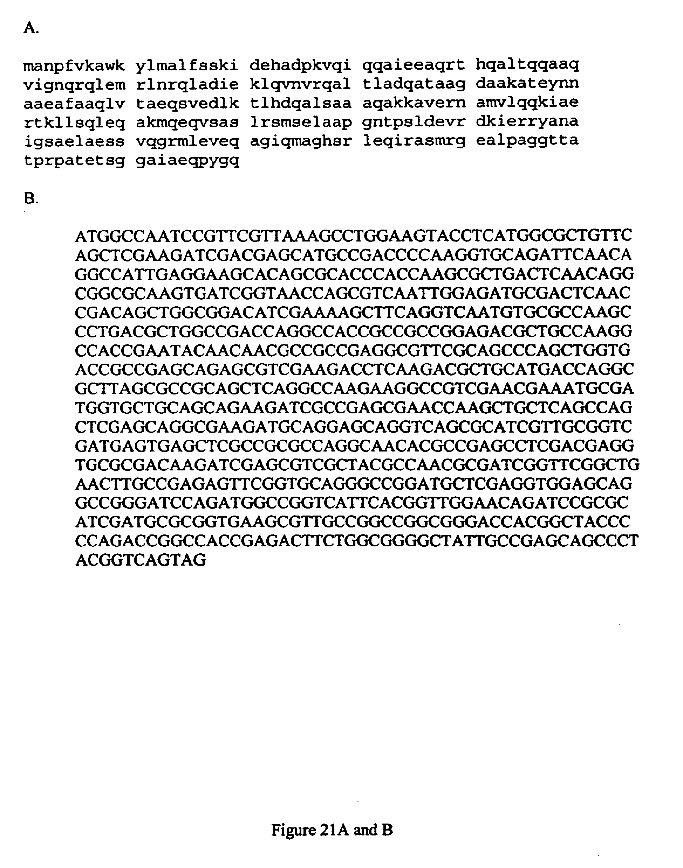

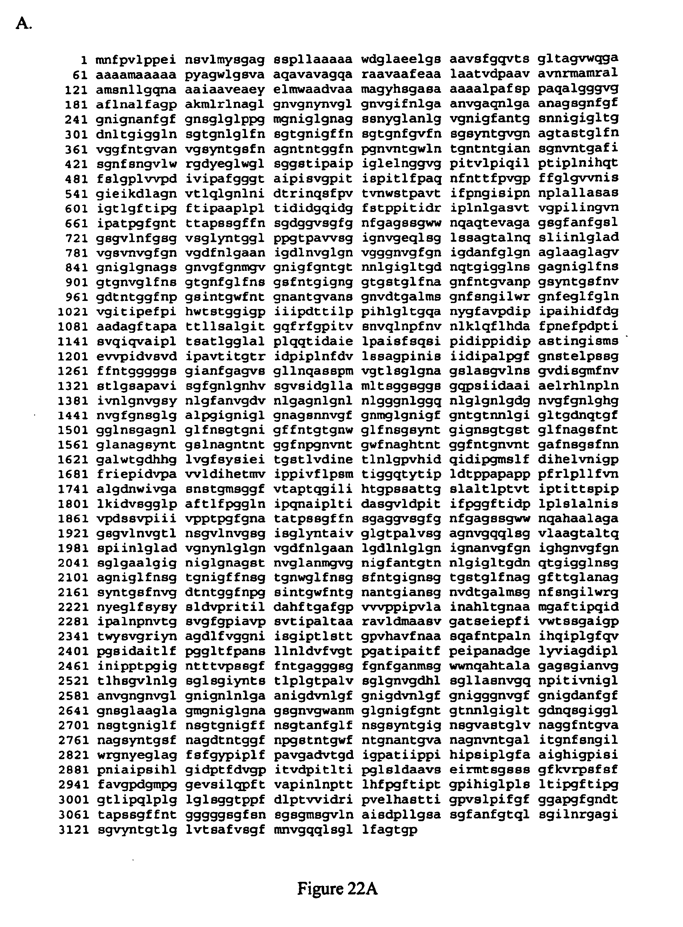

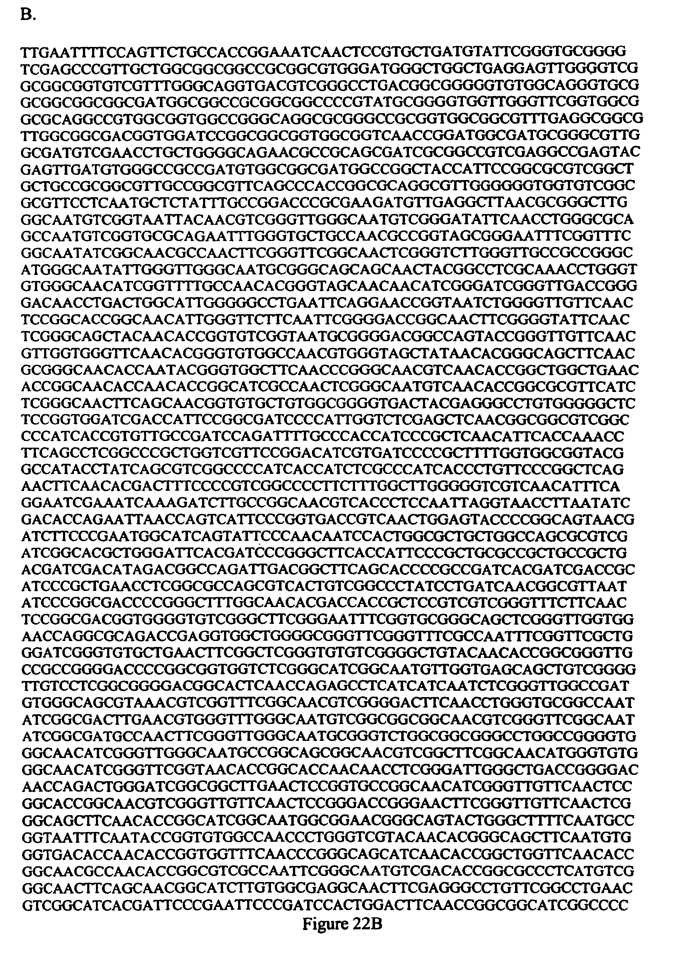

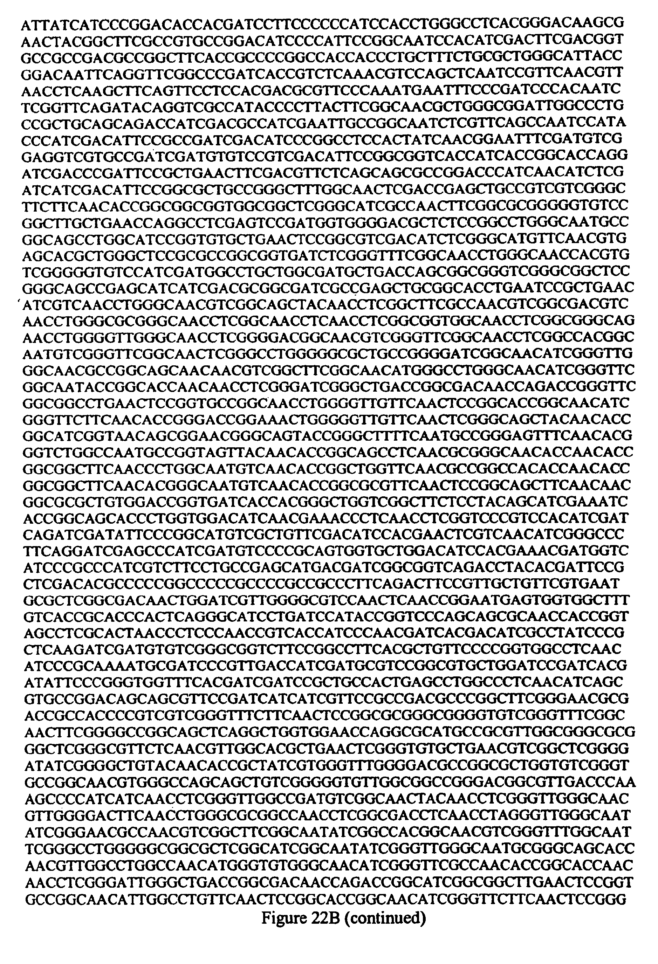

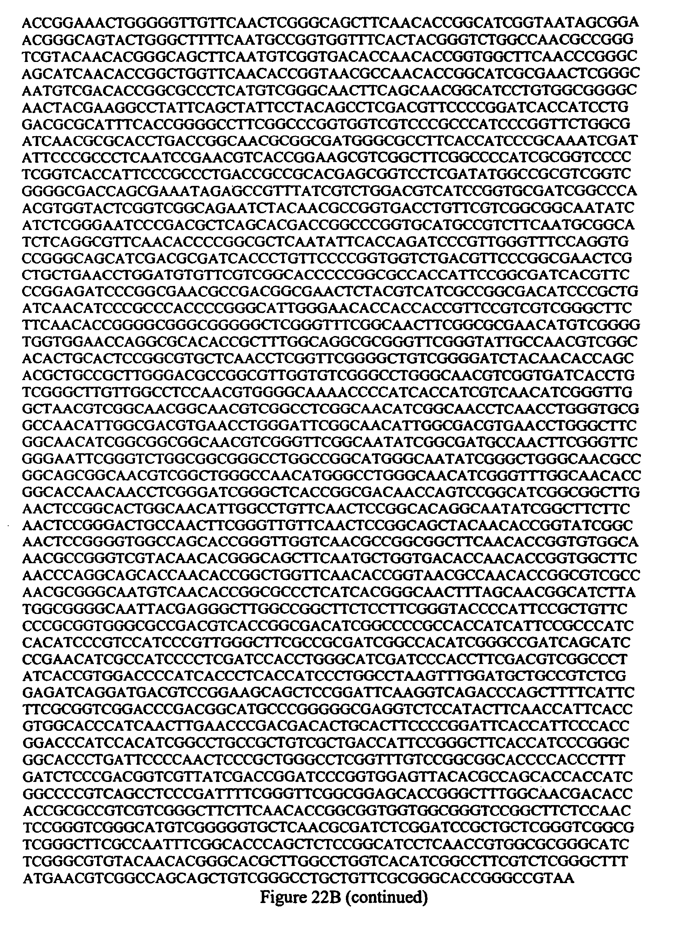

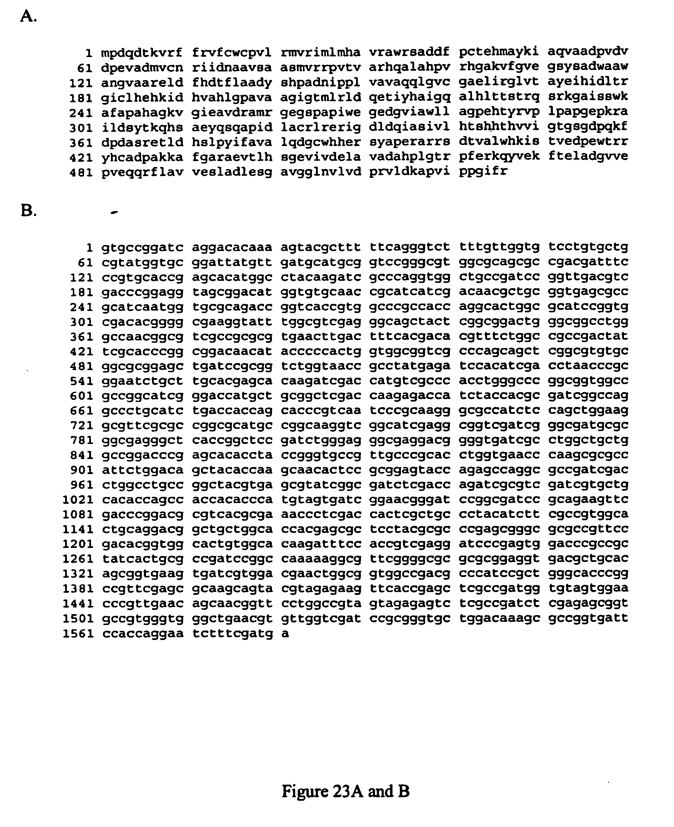

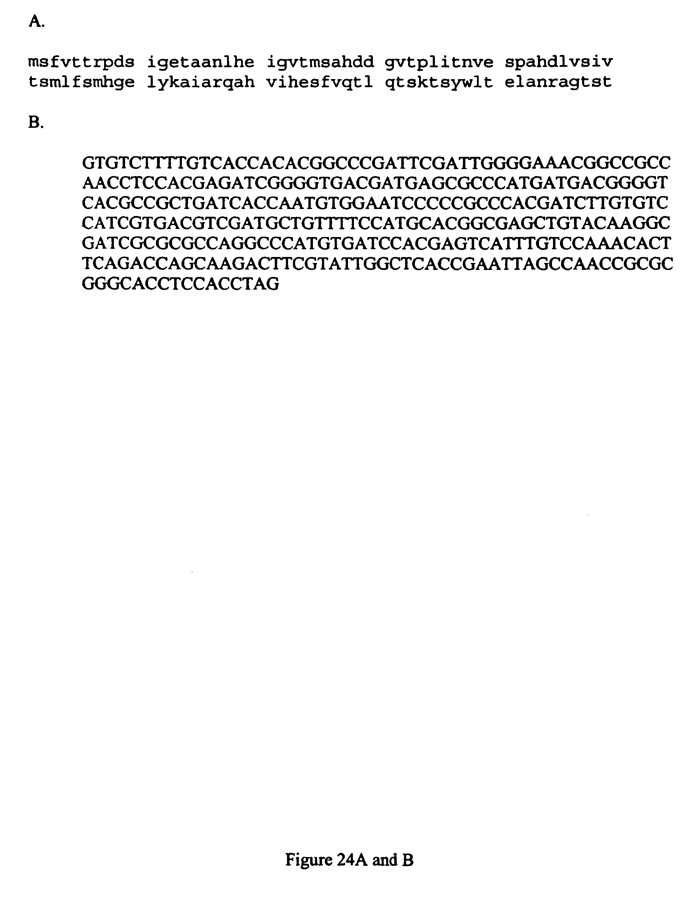

In general, at least one such antigen will be overexpressed and several different antigens may be overexpressed. For example, about 1-20 or more of such antigens, or alternatively about 19, 18, 17, 16, 15, 14, 13, 12, 11, 10, 9, 8, 7, 6, 5, 4, 3, 2, or only 1 of such antigens will be overexpressed by the rBCG. Further, multiple copies of one or more of the antigens may be encoded and overexpressed in the rBCG. The amino acid sequences of selected antigens, and the nucleic acid sequences that encode them, are depicted in Figures 5A-B to 25A-B.

In addition, the rBCG contains nucleic acid sequences comprising one or more genes that encodes and overexpresses at least one Mtb resuscitation/reactivation antigen. Those of skill in the art will be aware that the precise definition of "resuscitation" and "reactivation" antigens may vary somewhat within the field, and in some cases, the definitions may overlap. For the purposes of the invention, it is not necessary to separate resuscitation and reactivation antigens as all are identified as being of significance in the outgrowth of Mtb from the latent state. In the most accurate sense, resuscitation antigens (i.e. resuscitation promoting factors) are a subset of reactivation antigens defined as having significant sequence or functional homology to the resuscitation promoting factor of *Micrococcus luteus* (G Mukamolova et al, Archives of Microbiology, Volume 172, 1999). For the purposes of the present application, a "reactivation" antigen is a protein expressed by *M. tuberculosis* which elicits an immune response in humans with active tuberculosis but not latent Mtb infection. They may also be identified as immunogens expressed by Mtb during outgrowth from a non-replicative stationary phase in the Wayne model of latent tuberculosis. This may include molecules that are expressed during the emergence of Mtb from the dormant latent state into active tubercle bacilli. Examples of suitable reactivation/resuscitation antigens include Rv0867c, Rv0288, Rv1009, Rv0685, Rv0824c, Rv2744c, Rv3347c, Rv1130, Rv1169c, Rv1009, Rv1884c, Rv2389c, and Rv2450c. In a preferred embodiment, the reactivation/resuscitation antigens expressed are Rv0867c, Rv1884c, and Rv2389c.

In addition, in the rBCG of the invention the genes of the DosR (Dormancy Survival

Regulator) regulon, or a portion thereof, are upregulated and expressed. The entire regulon may be upregulated, or a suitable portion thereof. For example, genes that encode antigens that are recognized by individuals with latent TB may be the most suitable for upregulation. Examples of DosR upregulated antigens include Rev1738, Rv2623, Rv2031c, Rv2032, Rv2626c, Rv2005c, Rv3127, Rv1733c, Rv1996, Rv2628, Rv0079, Rv3130c, Rv3131, Rv1813c, Rv2006, Rv2029c, Rv2627c, Rv2030c, Rv3132c, and Rv2629. It is noteworthy that some overlap exists between latency and reactivation antigens, possibly reflecting the extended presence of latency related antigens in previously dormant organisms re-entering an active growth phase or their function in both the dormant and actively replicating state after reactivation in the mammalian host. Antigens that may be considered to overlap between latency and reactivation are listed in Table 4.

**Table 4. Antigens that overlap latency and reactivation**

| **Reactivation/dosR Antigens** | | | | | |
|---|---|---|---|---|---|
| Rv1996 | Rv2005 | Rv2029 | Rv2623 | Rv2626 | Rv2727 |

By "up-regulate" we mean that expression of each of the individual genes of the regulon or their translated proteins is increased above the level at which they are expressed when the regulon is in a "repressed" state. Proteins of the DosR regulon are normally expressed at a relatively low level. Upon oxygen starvation and/or the presence of oxidative nitrogen compounds the DosS and DosT proteins of TB complex organisms autophosphorylate and transfer this phosphate to DosR. DosR then binds to discrete sequences upstream of DosR regulated genes thereby activating their transcription and upregulating this group of genes and gene products which constitute the DosR regulon. Upregulation within the practice of this invention mimics this oxygen starvation effect where the DosR genes have increased transcription.

Those of skill in the art are familiar with approaches to genetically engineering an organism in order to up-regulate selected genes of interest, or selected regulons of interest. Such approaches include but are not limited to overexpression of the regulator, introduction of mutations in the regulator or sensor which render them constitutively active, the introduction of regulators which mimic the function of the regulator in question, introduction of kinases or feedback loop products which activate the sensor or regulator, or the introduction of genes/gene products which mimic the environmental state which causes activation of the sensor or regulator. In a preferred embodiment, the DosR regulon is up-regulated by over expressing the response regulator DosR (Rv3133c) of the DosRST "two component" regulatory system.

In another preferred embodiment, the vaccine includes one or more of Rv1908, Rv3873, Rv2780 and Rv1349. These are immunopotent antigens which were identified in silico and/or by experimentation.

In addition, the rBCG of the invention may encode antigens selected based on other criteria, such as demonstrated protective efficacy in an animal model or the expression of the antigen by Mtb but not BCG (J Mattow et al., Electrophoresis, 22:2936-2946, 2001, P.R. Jungblut, Molecular Microbiology, 33:1103-1117, 1999, H.J. Mollenkopf et al, Infection and Immunity, 72:6471-6479, 2004). In a preferred embodiment, the rBCG of the invention expresses Rv3407, which is normally expressed by Mtb but not BCG and has been shown to protect against tuberculosis in a mouse model.

The BCG that is genetically engineered as described herein may be of any BCG strain considered suitable, including but not limited to BCG strains BCG₁₃₃₁, BCG Pasteur, BCG Tokyo, BCG Copenhagen, BCG Moreau, or BCG Moscow.

In a preferred embodiment, the strain is BCG₁₃₃₁. In addition, the rBCG may be further genetically engineered to possess other traits, for example: a perfringolysin O (*pfo*) gene (in order to facilitate escape from the endosome); to express various selection markers such as antibiotic resistance or an auxotrophic selection marker in which a gene critical to the rBCG (e.g. for leucine or lysine synthesis) is deleted and must be complemented (e.g. by an extrachromsomal element that encodes the missing crucial gene) in order for the bacterium to survive; by deletion of genes or inhibition of the function of gene products which suppress apoptosis, etc.

In general, the rBCG of the invention is genetically engineered to overexpress selected antigens by introduction of genes encoding the antigens of interest into the chromosome of the rBCG under the transcriptional control of highly active expression control sequences, which may include those which are most active during mammalian infection. However, the genes encoding the antigens of interest could also be expressed from an extrachromosomal plasmid under the transcriptional control of highly active expression control sequences. Expression control sequences include but are not limited to promoters, ribosomal entry sites, etc.

The present invention further provides compositions for use in eliciting an immune response in and/or vaccinating a mammal. The compositions may be utilized as a vaccine against Mtb. The compositions of the invention include genetically engineered rBCG as described herein, and a pharmacologically suitable carrier. The preparation of such compositions for use as vaccines is well known to those of skill in the art. Typically, such compositions are prepared either as liquid solutions or suspensions, however solid forms such as tablets, pills, powders and the like are also contemplated. Solid forms suitable for solution in, or suspension in, liquids prior to administration may also be prepared. The preparation may also be emulsified. The active ingredients may be mixed with excipients which are pharmaceutically acceptable and compatible with the active ingredients. Suitable excipients are, for example, water, saline, dextrose, glycerol, ethanol and the like, or combinations thereof. In addition, the composition may contain minor amounts of auxiliary substances such as wetting or emulsifying agents, pH buffering agents, and the like. In addition, the composition may contain other adjuvants. If it is desired to administer an oral form of the composition, various thickeners, flavorings, diluents, emulsifiers, dispersing aids or binders and the like may be added. The composition of the present invention may contain any such additional ingredients so as to provide the composition in a form suitable for administration. The final amount of rBCG in the formulations may vary. However, in general, the amount in the formulations will be from about 1-99%. The compositions may further comprise additional adjuvants, suitable examples of which include but are not limited to Seppic, Quil A, Alhydrogel, etc.

Vaccine formulation also involves studies to determine maximum bacterial viability and stability throughout the manufacturing process. This includes determination of maximum organism viability (live to dead) during culture utilizing a variety of commonly used medium for the culture of Mycobacteria to include the addition of glycerol, sugars, amino acids, and detergents or salts. After culture cells are harvested by centrifugation or tangential flow filtration and resuspended in a stabilizing medium that allows for protection of cells during freezing or freeze-drying process. Commonly used stabilizing agents include sodium glutamate, amino acids or amino acid derivatives, glycerol, sugars or commonly used salts. The final formulation will provide sufficient viable organisms to be delivered by intradermal, percutaneous injection, perfusion or oral delivery with sufficient stability to maintain and adequate shelf life for distribution and use.

The methods of the present invention involve administering a composition comprising the rBCG of the invention in a pharmacologically acceptable carrier to a subject, usually a human mammal. The vaccine preparations of the present invention may be administered by any of the many suitable means which are well known to those of skill in the art, including but not limited to by injection, orally, intranasally, by ingestion of a food product containing the antigen, etc. However, in a preferred embodiment, the mode of administration is intradermal injection. In addition, the compositions may be administered alone or in combination with other medicaments or immunogenic compositions, e.g. as part of a multicomponent vaccine. Further, administration may be a single event, or multiple booster doses may be administered at various timed intervals to augment the immune response. In one embodiment, the vaccine preparation of the invention is used for an initial immunization, and the individual receiving the initial vaccine is then "boosted" with one or more different vaccine compositions, e.g. a known attenuated BCG vaccine. Alternatively, an individual may be vaccinated with another vaccine preparation and boosted one or more times with the vaccine preparation of the present invention. In some embodiments of the invention, the boosting compositions include nucleic acids encoding one or more Mtb antigens, for example: i) one or more antigens such as Rv1738, Rv2623, Rv2031c, Rv2032, Rv2626c, Rv2005c, Rv3127, Rv1733c, Rv1996, Rv2628, Rv0079, Rv3130c, Rv3131, Rv1813c, Rv2006, Rv2029c, Rv2627c, Rv2030c, Rv2629, Rv2450c, Rv1009, Rv0867c, Rv2389c, Rv1884c, Rv0288, Rv0685, Rv0824c, Rv2744, Rv3347c, Rv1130, Rv1169c, Rv1886, Rv1980c, Rv3804c, Rv3875, Rv1926c, Rv0467, Rv3873, Rv1908c, Rv1174c, Rv2780, Rv2620c, Rv1793, Rv1349 and Rv3132; ii) one or more antigens such as Rv1996, Rv2005, Rv2029, Rv2623, Rv2626 and Rv2727; iii) one or more antigens such as Rv2626, Rv1738, Rv2623, Rv1733, Rv2032, Rv3131, Rv3127, Rv3130c, Rv3804c and Rv1886c; and iv) one or more antigens including at least one of a dosR antigen, a reactivation antigen and/or a resuscitation antigen e.g., one antigen from each stage (latency, reactivation and resuscitation) of the life cycle of *M. tuberculosis.*

A particular advantage of the vaccine preparation of the present invention is that administration may be prophylactic, i.e. before exposure to the bacteria has occurred, or is suspected to have occurred, or after the fact, i.e. after a known or suspected exposure, or therapeutically, e.g. after the occurrence of disease symptoms associated with bacterial infection. This is because antigens that are involved in Mtb life cycle or infection and disease processes such as resuscitation and reactivation are included in the vaccine. Thus, the vaccine is useful not only for preventing the initial establishment of an Mtb infection, but also for preventing the reactivation of a latent Mtb infection.

Prior to administration to humans as a vaccine, the genetically engineered rBCG strains of the present are tested according to methods that are well-known to those of skill in the art. For example, tests for toxicity, virulence, safety, etc. are carried out in suitable animal models, e.g. in mice, guinea pigs, etc., some of which are immunocompromised. The ability of the vaccine preparations to elicit an immune response is likewise typically tested in suitable animal models, e.g. mice, guinea pigs, etc. In addition, protection studies involving vaccination, boosting, and subsequent challenge with live Mtb may be carried out using suitable animal models such as mice, guinea pigs, and non-human primates. Finally, those of skill in the art are familiar with the arrangements for carrying out clinical trials in consenting humans, in order to test the efficacy of the vaccine preparations. For details, see, for example, United States patent application 20060121054 (Sun et al.) published June 8, 2006, and references cited therein.

By "eliciting an immune response" we mean that administration of the vaccine preparation of the present invention causes the synthesis of specific antibodies (at a titer in the range of I to 1 x 10⁶, preferably 1 x 10³, more preferable in the range of about 1 x 10³ to about 1 x 10⁶, and most preferably greater than 1 x 10⁶) and/or cellular proliferation, as measured, e.g. via cellular assays in which IFN-γ production is assessed, for example, by ³H thymidine incorporation, or other suitable means. In a preferred embodiment, the immune response is a protective immune response, i.e. the immune response protects the vaccinated individual from future challenge with Mtb. However, those of skill in the art will recognize that a vaccine or immunostimulating preparation need not provide total protection in order to convey some benefit to a patient. For example, the preparations may elicit immune responses that slow or lessen the progress of disease symptoms, without fully eradicating them.

The following non-limiting Examples serve to illustrate the practice of this invention.

### EXAMPLES

### EXAMPLE 1. Selection of suitable antigens and design of rBCG based on this selection

An initial group of Mtb proteins (189 antigens, see Table 1) was selected from all possible 3989 Mtb ORFs, according to the following selection procedure:
(1) Compilation of available data for all 3989 TB ORF products: Literature scan for global analyses (e.g. up/down regulation of expression under various conditions such as hypoxia, dormancy, interaction with macrophage, location and persistence in lung tissue; iron regulation; transcriptomics and proteomics profiles; mutation leading to attenuation of virulence; potential high immune response etc.).
(2) Establishing a subset of genes by cross matching the accumulated data: Selection of antigens that demonstrate positive evidence in any two independent studies originating from different criteria mentioned above.
(3) Iterative trimming of the above subset, aiming at selecting an initial group of candidates (Table 1, 189 antigens), based on the extent of effect documented in the individual studies.
(4) Conducting bioinformatic studies for:
   a) Further characterization of the initial group of 189 candidates by domain analyses, prediction of cellular localization, inspection of genome context, identification of repeat proteins, and assignment of putative function for the unknown/hypothetical Ag (sequence similarity searches by blast against the nr database, NCBI, domain/motifs databases, and Mtb-related servers and databases: TB-sgc - The TB Structural Genomics Consortium (Web site located at www.doe-mbi.ucla.edu/TB/), Tuberculist- the database on *Mycobacterium tuberculosis* genetics (Web site located at genolist.pasteur.fr/TubercuList/), TBDB - an integrated platform for TB drug discovery (Web site located at www.tbdb.org) MTBreg - The Database of Conditionally Regulated Proteins in *Mycobacterium tuberculosis* (Web site located at www.doe-mbi.ucla.edu/Services/MTBreg/) and the BioHealthBase server (Web site located at www.biohealthbase.org/GSearch).
   b) Immunoinformatic analysis for prediction of human leukocyte antigen (HLA)-binding targets (by NetCTL) and experimentally documented T-cell epitopes by database (IEDB) and literature searches. Implementing various measures for the determination of epitopes as putative strong binders, and for the selection of most potent candidates according to the predictions (i.e. thresholds, and number of supertypes/population coverage).
(5) In depth literature search for data pertaining to aspects of virulence and vaccine development, for each of the 189 antigens (Table 1) and/or their orthologs.
(6) Setting up a knowledge dataset of the 189 candidates (Table 1), by integrating all literature and data analyses (steps 4&5 above).

The selected antigens in the initial group presented in Table 1, include known vaccine candidates, and proteins known to be: involved in various stages of the life cycle of Mtb (dormancy, reactivation, resuscitation); tissue specific antigens; antigens affected by starvation; and, antigens originating in genomic sequences present in a virulent Mtb strain.

The 189 candidate antigens were ranked in order of likely importance by the following 14 analyses:
1) macrophage survival/persistence;
2) up-regulation of expression by the two-component system MprAB;
3) response to hypoxia;
4) involvement in reactivation;
5) involvement in dormancy;
6) expression in lung tissue;
7) co-regulation with Rv2031 (Acr);
8) whether or not the protein is secreted;
9) the ability to act as a B-cell immunogen;
10) iron-regulated genes;
11) cell wall association or cell wall biogenesis;
12) existing vaccine efficacy data;
13) the presence of repeat domains;
14) T-cell response, determined by a) known experimental evidence (e.g. from the literature) and b) a determination that the antigen likely contains one or more T-cell epitopes as assessed by the immunoinformatics program of the Technical University of Denmark (cbs.dtu.dk/services/NetCTL);

The presence or absence of each of these traits was scored for each of the 189 genes and a qualitative score was determined and employed as a measure to rank the list of 189 antigens and choose for the 45 best hits.

The 45 candidates were then further ranked using the same 13 criteria, by assigning internal numerical scores to each of the criteria, according to the intensity of the results and/or relevance to vaccine development. A list of the top-ranking 45 antigens is given in Table 2, along with their subgrouping according to the class/phase of infection (latency/dormancy, resuscitation/reactivation, classical and others); within the classes, the antigens are sorted by their scores. Table 3 presents the 45 antigens prioritization into 3 subset groups according to their quantitative, and subsequently - qualitative scores.

Based on this analysis, final selections of groups of antigens for use in the rBCGs were made, usually based on the antigens with the highest overall scores. In addition, for the final selection, antigens were grouped according to "type" in that the rBCG includes at least 1) one or more Mtb antigens, including the so-called "classical" Mtb antigens such as 85A, 85B and TB 10.4; and 2) at least one Mtb resuscitation/reactivation antigen selected from Rv0867c, Rv1009, Rv1884c, Rv2389c, Rv2450c, Rv0867c, Rv0288, Rv1009, Rv0685, Rv0824c, Rv1349, Rv2744c, Rv3347c, Rv1130, and Rv1169c.

In addition, antigens were selected based on other criteria such as demonstrated protective efficacy in an animal model, the expression of the antigen by Mtb but not BCG, or diminished expression of the antigen in BCG (J Mattow et al., Electrophoresis, 22:2936-2946,2001, P.R. Jungblut, Molecular Microbiology, 33:1103-1117,1999, H.J. Mollenkopf et al, Infection and Immunity, 72:6471-6479). Such Mtb-specific antigens include Rv1511, Rv2520c, Rv3407, Rv2802c and Rv3710.

Preferred combinations of antigens to be expressed in an rBCG include the following:
1) Classical antigens Rv1886c, Rv3804c;
2) Resuscitation and Reactivation antigens Rv0867c, Rv1884c, Rv2389c; and
3) Mtb-specific antigen Rv3407.

### EXAMPLE 2. Construction of a recombinant BCG genetically engineered to express at least one classical Mtb antigen, at least one Mtb resuscitation/reactivation antigen and in which the DosR regulon is upregulated.

### Materials and Methods: General

For the construction of an rBCG described in the following sections, restriction endonucleases (herein "REs"); New England Biolabs Beverly, MA), T4 DNA ligase (New England Biolabs, Beverly, MA) and Taq polymerase (Invitrogen, Carlsbad, CA) were used according to the manufacturers' protocols; Plasmid DNA was prepared using small-scale (Qiagen MiniprepR kit, Santa Clara, CA) or large-scale (Qiagen MaxiprepR kit, Santa Clara, CA) plasmids DNA purification kits according to the manufacturer's protocols (Qiagen, Santa Clara, CA); Nuclease-free, molecular biology grade Milli-Q water, Tris-HCl (pH 7.5), EDTA pH 8.0, 1M MgCl⁻², 100% (v/v) ethanol, ultra-pure agarose, and agarose gel electrophoresis buffer were purchased from Invitrogen, Carlsbad, CA. RE digestions, PCRs, DNA ligation reactions and agarose gel electrophoresis were conducted according to well-known procedures (Sambrook, et al., Molecular Cloning: A Laboratory Manual. 1, 2, 3; 1989); (Straus, et al., Proc Natl Acad Sci USA. Mar; 87(5): 1889-93; 1990). Nucleotide sequencing to verify the DNA sequence of each recombinant plasmid described in the following sections was accomplished by conventional automated DNA sequencing techniques using an Applied Biosystems automated sequencer, model 373A.

PCR primers were purchased from commercial vendors such as Sigma (St. Louis, MO) or synthesized using an Applied Biosystems DNA synthesizer (model 373A). PCR primers were used at a concentration of 150-250 µM and annealing temperatures for the PCR reactions were determined using Clone manager software version 4.1 (Scientific and Educational Software Inc., Durham, NC). PCRs were conducted in a BioRad thermocycler (BioRad, Hercules, CA). The PCR primers for the amplifications were designed using Clone Manager^{®} software version 4.1 (Scientific and Educational Software Inc., Durham NC). The RE digestions and the PCRs were subsequently analyzed by agarose gel electrophoresis using standard procedures (Straus et al, supra 1990; and Sambrook et al., supra 1989). A positive clone is defined as one that displays the appropriate RE pattern and/or PCR pattern. Plasmids identified through this procedure were further evaluated using standard DNA sequencing procedures, as described above.

*Escherichia coli* strains, such as DH5α and Stable2^{R}, were purchased from Invitrogen (Carlsbad, CA) and served as initial host of the recombinant plasmids. Recombinant plasmids were introduced into *E*. *coli* strains by electroporation using a high-voltage eletropulse device, such as the Gene Pulser (BioRad Laboratories, Hercules, CA), set at 100-200Ω 15-25µF and 1.0-2.5 kV, as described (Straus et al, supra 1990). Optimal electroporation conditions were identified by determining settings that resulted in maximum transformation rates per mcg DNA per bacterium.

*E. coli* strains are typically grown on tryptic soy agar (Difco, Detroit, MI) or in tryptic soy broth (Difco, Detroit, MI), which was made according to the manufacturer's directions. Unless stated otherwise, all bacteria were grown at 37°C in 5% (v/v) CO₂ with gentle agitation. When appropriate, the media was supplemented with antibiotics (Sigma, St. Louis, MO). Bacterial strains were typically stored at -80°C suspended in (Difco) containing 30% (v/v) glycerol (Sigma, St. Louis, MO) at ca. 10⁹ colony-forming units (herein referred to as "cfu") per ml.

Mycobacterial strains were cultured in liquid media, such as Middlebrook 7H9 or Saulton Synthetic Medium, preferably at 37°C. The strains can be maintained as static or agitated cultures. In addition, the growth rate of BCG can be enhanced by the addition of oleic acid (0.06% v/v; Research Diagnostics Cat. No. 01257) and detergents such as Tyloxapol (0.05% v/v; Research Diagnostics Cat. No. 70400). The purity of BCG cultures can be evaluated by evenly spreading 100 mcl aliquots of the BCG culture serially diluted (e.g. 10-fold steps from Neat - 10⁻⁸) in phosphate buffered saline (herein referred to PBS) onto 3.5 inch plates containing 25-30 ml of solid media, such as Middlebrook 7H10. In addition, the purity of the culture can be further assessed using commercially available medium such as thioglycolate medium (Science Lab, catalogue number 1891) and soybeancasein medium (BD, catalogue number 211768).

In order to insert the desired antigen expression cassettes into the chromosome of the perfringolysin expressing BCG1331 derivative described elsewhere, a plasmid was designed in silico and synthesized by DNA2.0 (Menlo Park, CA). The salient features of this vector (pJFINT) include an *E.coli* colE1 origin of replication, 3 multiple cloning sites separated by transcriptional terminators rmBT1, T2 of pEX18gm, and mhA, the attP phage integration region of bacteriophage L5 and the integrase gene of bacteriophage L5 (GenBank #Z18946). Immediately upstream of the L5 sequence, a selectable marker cassette consisting of a kanamycin resistance allele *aphA* from Tn10 (GenBank #AAM97345) and a *sacB* gene (Genbank # NP_391325) were included. This marker cassette was flanked by direct repeats of the γA resolvase binding site from transposon Tn1000. This plasmid is incapable of replication in mycobacterial species and the L5 attP sequence allows for high frequency recombination with the attB region of mycobacterial chromosomes to facilitate integration of the plasmid sequence into the chromosome. The marker cassette can then be removed from the chromosome of the integrant by the introduction of γΔ resolvase and selection of markerless strains on solid media containing 10% sucrose.

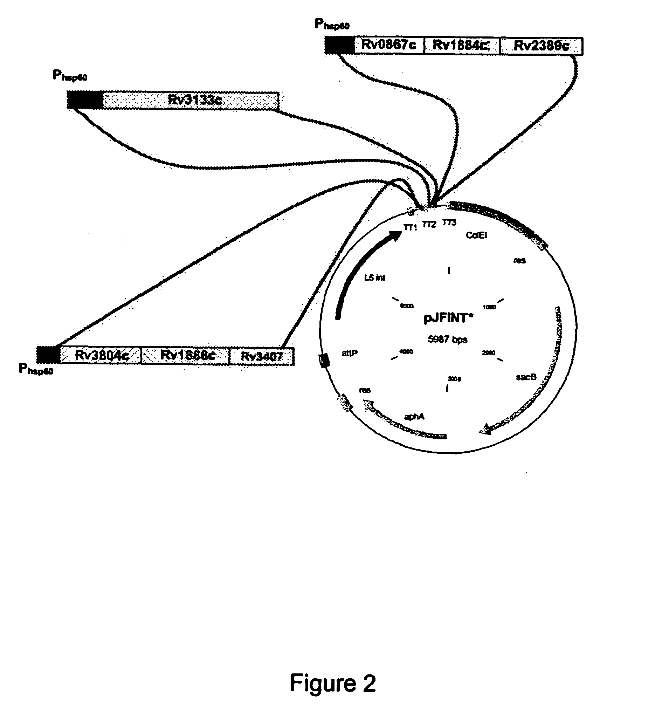

An antigen expression cassette was designed in silico to encode Rv0867c, Rv1884c, and Rv2389c separated by optimized ribosomal sites under the transcriptional control of the *hsp60* promoter of *Mycobacterium bovis* and synthesized by DNA2.0 (Carlsbad CA). A second expression cassette was similarly designed and constructed to encode Rv1886c, Rv3804c and Rv3407c under transcriptional control of the *hsp60* promoter. Finally, a third cassette encoding Rv3133c (DosR) under the transcriptional control of the hsp60 promoter was designed and constructed. These three antigen expression cassettes were ligated into pJFINT such that each was separated by a transcriptional terminator (Figure 2). The resulting plasmid, pRC 108, was electroporated into *E*. *coli* Stable2 and the plasmid sequence of a kanamycin resistant clone was verified.

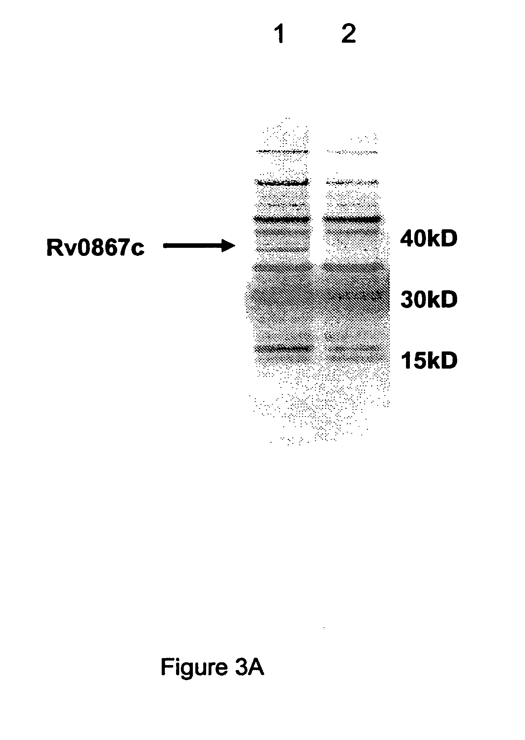

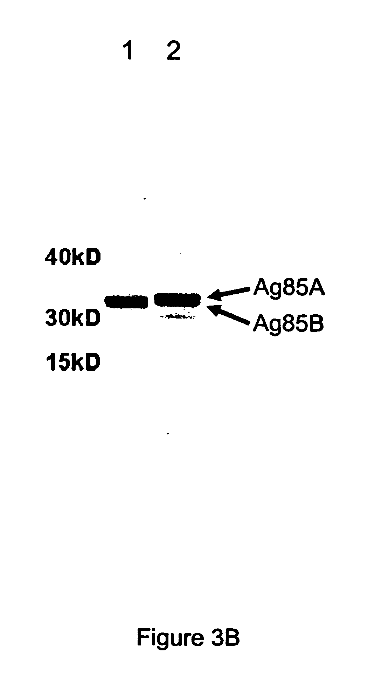

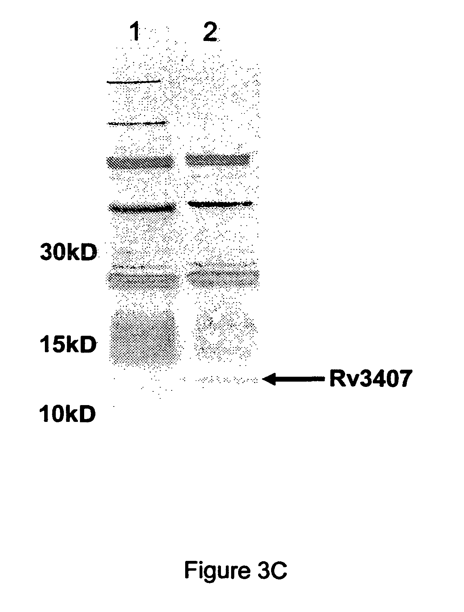

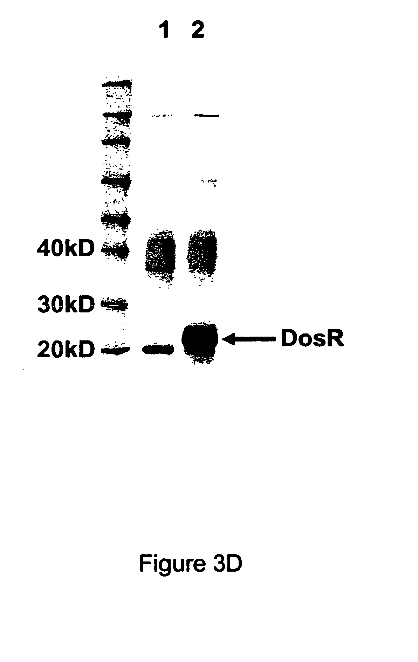

The pRC 108 plasmid was isolated from a 100 ml *E*. *coli* culture and electroporated into a *pfo* expressing derivative of BCG Danish 1331. After electroporation, the cells were cultured overnight in 7H9 medium with 10% (v/v) OADC and 0.05% (v/v) of Tyloxapol supplementation and plated on 7H 10 agar containing 50 ug/ml of kanamycin. As the plasmid does not encode a mycobacterial origin of replication, kanamycin resistance in all colonies tested was conferred by integration of the plasmid into the attB site of the BCG genome. Individual colonies were picked for PCR analysis and inoculated into 7H9 medium with 10% (v/v) OADC and 0.05% (v/v) tyloxapol for analysis of antigen expression. PCR characterization of the kanamycin resistant colonies demonstrated the presence of the entire plasmid sequence in the chromosome of the recombinant BCG, designated AFV-102pRC108. AFV-102pRC108 cultures were washed with 7H9 and used to inoculate proteins-free 7H9tyloxapol cultures. Supernatants of the AFV-102pRC108 cultures were harvested by centrifugation and immunoblotted with rabbit polyclonal antisera to the transglycolase domain of the Rpf protein. Rv1009, which is cross reactive with the transglycosylase domains of all Rpf's or rabbit polyclonal antisera raised against peptides of Rv0867c. This demonstrated the enhanced production of Rv0867c, Rv1884c, and Rv2389c above the background of the BCG homologs of these proteins (Figure 3A). This supernatant was similarly immunoblotted with rabbit polyclonal antisera to Ag85complex and the overexpression of Rv1886c and Rv3804c was observed (Figure 3B). The cell pellet was immunoblotted with rabbit polyclonal antisera to Rv3407 and the expression of Rv3407, the homolog of which is normally silent in BCG, was confirmed (Figure 3C). Cell pellets were also immunoblotted with DosR-specific rabbit polyclonal antisera to demonstrate the overexpression of DosR itself and with rabbit antisera to Rv1733c to demonstrate the upregulation of a DosR regulated protein (Figure 3D).

In order to complete the construction of this vaccine to make it suitable for human use, the marker cassette of the integrated plasmid was then removed. Electrocompetent AFV-102pRC108 cells were electroporated with plasmid pYUB870hyg, which encodes the γΔ resolvase of Tn1000, a *sacB* allele, and a hygromycin resistance gene (GenBank #ABD64366). Transformants resistant to both kanamycin and hygromycin were selected on 7H10 media and inoculated into 7H9 liquid media with 10% (v/v) OADC and 0.05% (v/v) tyloxapol and no antibiotics. After seven days growth, dilutions of these liquid cultures were plated on 7H10 containing 10% sucrose to select for recombinants from which the *aphA-sacB* marker has been excised and the pYUB870hyg plasmid has been lost by dilution and selection against the *sacB* allele.

Sucrose-resistant transformants were picked for PCR analysis of the integrated antigen cassettes and were inoculated into 7H9 liquid media for immunoblot analysis as before. PCR analysis revealed that the antigen expression cassettes were still present in the chromosome and that the hygromycin resistance marker and *sacb* gene had been excised. Immunoblotting of supernatants and cell pellets with antisera to Rpf, Ag85 complex, Rv3407, and DosR confirmed that excision of the marker cassette from the chromosome did not effect the expression of the inserted antigen cassettes.

Strain AERAS-407 was constructed by removal of the anti-biotic resistance marker from AFV-102pRC108.

### EXAMPLE 3. Demonstration of the immunogenicity and protection elicited by vaccination with AERAS-407 in mice

To illustrate the immune responses elicited by AERAS-407 and to demonstrate its protective efficacy against tuberculosis, four groups of 10 C57/BL6 mice are vaccinated subcutaneously with either 1) saline, 2)BCG, 3)BCG-PfoA or 4)AERAS-407. Mice in groups 2, 3, and 4 receive 5x10⁵ cfu of BCG or recombinant BCG. After 10 weeks, five mice from each group are sacrificed for immune assays and the remaining animals are challenged with 100 cfu aerosolized *M. tuberculosis* Erdman.

To measure humoral immune responses, a peptide microarray chip has been designed. The chip is spotted with overlapping 50 amino acid peptides generated from the sequences of Rv1886c, Rv3804c, Rv3407c, Rv0867c, Rv1884c, Rv2389c, Rv3133c, and all BCG encoded DosR-regulated proteins which are at least 2 fold induced by the overexpression of DosR (i.e. DosR regulated genes for which transcription is at least 2 fold greater in BCG constitutively expressing the DosR regulon than the wild-type parent strains under aerobic conditions). Pooled sera from each group is incubated with 3 peptide chips for 1 hr at 37°C. The chips are then washed with phosphate-buffered saline (PBS) pH7.2 and incubated with fluorescein isothiocyanate (FITC) conjugated goat anti-mouse IgG sera (Abcam, Cambridge, MA) for 1 hour at 37°C. The chips are then washed again with PBS and the immunofluorescence is read with a Genepix 4000B Array Scanner (Molecular Devices, Sunnyvale, CA). Values for each peptide spot are averaged for the 3 chips in each group. Mice receiving AERAS-407 show greater antibody responses to peptides derived from Rv1886c, Rv3804c, Rv3407c, Rv0867c, Rv1884c, Rv2389c and DosR regulated proteins than unvaccinated mice or mice vaccinated with BCG or BCG-PfoA.

To measure cellular immune responses elicited by AERAS-407, spleens are harvested from each and homogenized. Splenocyte concentrations are adjusted to 5x10⁵ cells/well in R10 media in multichamber plates and are incubated for 3 days at 37°C with purified Rv1886c, Rv3804c, Rv3407c, Rv0867c, Rv1884c, or Rv2389c at a concentration of 1µg/ml. In addition, splenocytes from each mouse are incubated with Rv2623 and Rv3130c as these proteins are known to be highly upregulated in DosR overexpressing strains and are known to be potent T cell immunogens. After the 3 day incubation, supernatants are harvested from the splenocyte cultures and assayed by ELISA for interferon-γ produced in response to antigen stimulation. Control cultures of unstimulated splenocytes or PMA/PHA stimulated splenocytes are included as negative and positive controls, respectively.

To quantify protection afforded by vaccination with AERAS-407, the 5 remaining mice from each group are sacrificed 10 weeks after challenge with *M. tuberculosis* and lungs and spleens are aseptically harvested from each animal. Lungs and spleens are homogenized in PBS and dilutions are plated on 7H 10 agar. After 4 weeks of incubation, the number of *M*. *tuberculosis* colonies are enumerated for each animals lungs and spleen and corrected for the dilution factor. This value is the number of live tubercle bacilli present in the lungs and spleen of each animal. BCG vaccination typically results in approximately a 1 log reduction in cfu/lung and spleen versus saline vaccination. AERAS-407 vaccination results in a greater reduction of Mtb load in the lungs and spleens of mice.

### EXAMPLE 4. Construction of a second recombinant BCG vaccine genetically engineered to over express selected classical M. tb antigens and the DosR regulon

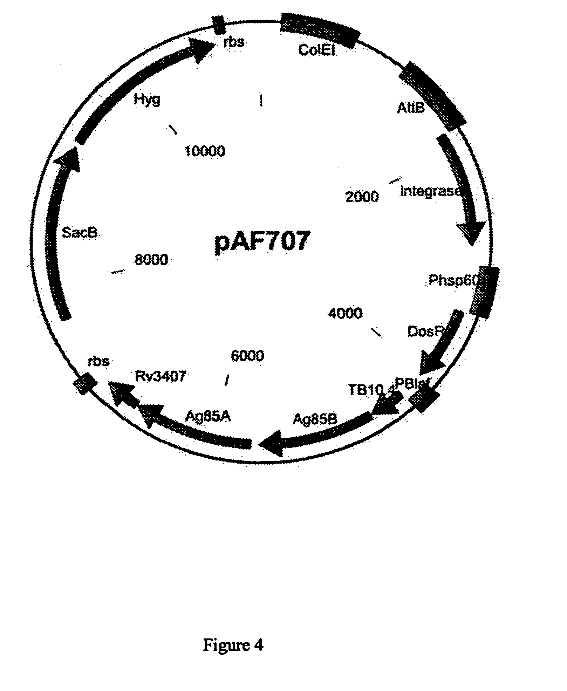

A second combination of antigens (TB 10.4, Ag85B, Ag85A and Rv3407) was also overexpressed in the rBCG AFV102 strain by integration into the chromosome at the attB site. This was carried out in a manner similar to that described in Example 2 for the Aeras 407 construct. To integrate this second antigen set, the integration plasmid pAF707 (depicted schematically in Figure 4) was constructed as described for pJFINT in Example 2 except for the following differences: a hygromycin resistance gene was used in place of the kanamycin resistance gene, and the PBlaf promoter was used to drive antigens TB10.4, Ag85B, Ag85A and Rv3407 for over expression in the order listed. All other general materials and methods were similar to those described in Example 2. The resultant strain was designated Aeras 406. The final strain was further analyzed by PCR followed by sequencing analysis and confirmed to have the desired genotype. The strain was further demonstrated to overexpress the selected antigens by SDS-PAGE analysis and immunoblot testing as described in Example 2 (data not shown).

### EXAMPLE 5. Protection of non-human primates from tuberculosis by vaccination with AERAS-407.

The protective efficacy of AERAS-407, particularly in relation to the enhanced capacity to prevent latent infection and reactivation of disease, is best tested in rhesus macaques as mice do not create granulomatous latent foci as humans and other primates do. To evaluate the protective efficacy of AERAS-407 in NHP's, four groups of six weight and sex-matched rhesus macaques are vaccinated with 1) saline, 2) BCG 1331, 3) BCG-Pfo, or 4) AERAS-407. Each animal in groups 2-4 receives 5x10⁵ cfu of the respective BCG or rBCG by intradermal injection. Fifteen weeks after vaccination, all animals are challenged by bronchial installation of approximately 300 cfu of *M. tuberculosis* Erdman. All animals are evaluated monthly for six months for clinical symptoms of tuberculosis by chest X-ray, weight, feeding, cough, lethargy, and immune responses to TB specific proteins. All animals that die during the six month observation period are necropsied and tissue pathology and Mtb burden by organ is measured as in Example 3. All moribund animals are humanely euthanized and similarly examined. Six months post-challenge all surviving animals are euthanized and necropsied for tissue pathology and Mtb burden in lungs, liver and spleen. AERAS-407 vaccination results in decreased mortality, decreased tissue damage and lower counts of viable Mtb organisms in the lungs of experimentally infected animals.

## Claims

1. A recombinant Bacille Calmette-Guerin (BCG) wherein at least one DosR regulon gene is up-regulated and said BCG comprises at least two nucleic acid sequences which are different from each other and each of which encodes one or more genes that are overexpressed, said at least two nucleic acid sequences including
i) a first nucleic acid sequence encoding at least one Mycobacterium tuberculosis (Mtb) antigen; and
ii) a second nucleic acid sequence encoding at least one Mtb resuscitation or Mtb reactivation antigen that is not a DosR antigen.

2. The recombinant BCG of claim 1, wherein one or more of said at least two nucleic acid sequences is integrated into a chromosome of said rBCG.

3. The recombinant BCG of claim 1, wherein one or more of said at least two nucleic acid sequences is an extrachromosomal nucleic acid sequence.

4. The recombinant BCG of claim 1, wherein said at least one Mtb antigen is 85A or said at least one Mtb antigen is 85B,

5. The recombinant BCG of claim 1, wherein said at least one Mtb resuscitation or reactivation antigen is selected from the group consisting of Rv0867c, Rv1009, Rv1884c, Rv2389c, Rv2450c, Rv0288, Rv0685, Rv0824c, Rv2744c, Rv3347c, Rv1130 and Rv1169c.

6. The recombinant BCG of claim 1, wherein said at least one Mycobacterium tuberculosis (Mtb) antigen is selected from the group consisting of Rv2623, Rv2626c, Rv2005c, Rv1996, Rv2029c, Rv2627c, Rv1908, Rv3837, Rv2780 and Rv1349.

7. The recombinant BCG of claim 1, wherein expression of said at least one DosR regulon gene is up-regulated by enhanced promoter activity.

8. A recombinant Bacille Calmette-Guerin (BCG) according to claim 1 comprising at least two nucleic acid sequences which are different from each other and each of which encode one or more genes that are overexpressed,
wherein at least a first nucleic acid sequence is selected from the group consisting of Rv2450c, Rv1009, Rv0867c, Rv2389c, Rv1884c, Rv0288, Rv0685, Rv0824c, Rv2744, Rv3347c, Rev1130, Rv1169c; and
wherein at least a second nucleic acid sequence is selected from the group consisting of Rv1738, Rv2623, Rv2031c, Rv2032, Rv2626c, Rv2005c, Rv3127, Rv1733c, Rv1996, Rv2628, Rv0079, Rv3130c, Rev3131, Rv1813c, Rv2006, Rv2029c, Rv2627c, Rv2030c, Rv2629, Rv2450c, Rv1009, Rv0867c, Rv2389c, Rv1884c, Rv0288, Rv0685, Rv0824c, Rv2744, Rv3347c, Rev1130, Rv1169c, Rv1886, Rv1980c, Rv3804c, Rv3875, Rv1926c, Rv0467, Rv3873, Rv1908c, Rv1174c, Rv2780, Rv2620c, Rv1793, Rv1349 and Rv3132; and
wherein at least one DosR regulon gene is up-regulated.

9. A vaccine composition comprising
a recombinant Bacille Calmette-Guerin (BCG) according to any one of claims 1 to 8 for use in immunizing a subject against infection Mtb or for eliciting an immune response to Mtb in said subject,
wherein said vaccine composition is administered in an amount sufficient to immunize said subject against infection by Mtb or to elicit an immune response to Mtb in said subject,

10. The vaccine composition of claim 9 for use according to claim 9, wherein the vaccine composition is adapted to be administered further as a boosting composition comprising one or more Mtb antigens.

11. The vaccine composition of claim 10 for use according to claim 10, wherein said one or more antigens is selected from the group consisting of Rv1738, Rv2623, Rv2031c, Rv2032, Rv2626c, Rv2005c, Rv3127, Rv1733c, Rv1996, Rv2628, Rv0079, Rv3130c, Rv3131, Rv1813c, Rv2006, Rv2029c, Rv2627c, Rv2030c, Rv2629, Rv2450c, Rv1009, Rv0867c, Rv2389c, Rv1884c, Rv0288, Rv0685, Rv0824c, Rv2744, Rv3347c, Rv1130, Rv1169c, Rv1886, Rv1980c, Rv3804c, Rv3875, Rv1926c, Rv0467, Rv3873, Rv1908c, Rv1174c, Rv2780, Rv2620c, Rv1793, Rv1349 and Rv3132.

12. The vaccine composition of claim 10 for use according to claim 10, wherein said one or more antigens is selected from the group consisting of Rv1996, Rv2005, Rv2029, Rv2623, Rv2626 and Rv2727 or wherein said one or more antigens is selected from the group consisting of Rv2626, Rv1738, Rv2623, Rv1733, Rv2032, Rv3131, Rv3127, Rv3130c, Rv3804c and Rv1886c.

13. A vaccine composition comprising
a recombinant Bacille Calmette-Gruerin (BCG) according to any one of claims 1 to 8 for use in preventing a recurrence of symptoms of tuberculosis in a patient with a latent Mtb infection,
wherein said vaccine composition is administered in an amount sufficient to prevent a recurrence of symptoms of tuberculosis in said patient.

14. The vaccine composition of claim 13 for use according to claim 13, further comprising the step of administering a boosting composition comprising nucleic acid sequences encoding one or more Mtb antigens.

15. The vaccine compositions of claim 14 for use according to claim 14, wherein said one or more antigens is selected from the group consisting of Rv1738, Rv2623, Rv2031c, Rv2032, Rv2626c, Rv2005c, Rv3127, Rv1733c, Rv1996, Rv2628, Rv0079, Rv3130c, Rv3131, Rv1813c, Rv2006, Rv2029c, Rv2627c, Rv2030c, Rv2629, Rv2450c, Rv1009, Rv0867c, Rv2389c, Rv1884c, Rv0288, Rv0685, Rv0824c, Rv2744, Rv3347c, Rv1130, Rv1169c, Rv1886, Rv1980c, Rv3804c, Rv3875, Rv1926c, Rv0467, Rv3873, Rv1908c, Rv1174c, Rv2780, Rv2620c, Rv1793, Rv1349 and Rv3132.

16. The vaccine composition of claim 14 for use according to claim 14, wherein said one or more antigens is selected from the group consisting of Rv1996, Rv2005, Rv2029, Rv2623, Rv2626 and Rv2727 or wherein said one or more antigens is selected from the group consisting of Rv2626, Rv1738, Rv2623, Rv1733, Rv2032, Rv3131, Rv3127, Rv3130c, Rv3804c and Rv1886c.

17. The vaccine composition of claim 10 for use according to claim 10, wherein said one or more antigens include at least one antigen from each stage of a life cycle of Mtb.

## Patentansprüche

1. Rekombinantes Bazillus Calmette-Guerin (BCG), worin mindestens ein DosR Regulon Gen heraufreguliert ist und das BCG mindestens zwei Nukleinsäuresequenzen umfasst, die voneinander unterschiedlich sind und jedes hiervon kodiert ein oder mehrere Gene, die überexprimiert sind, diese mindestens zwei Nukleinsäuresequenzen schließen ein
i) eine erste Nukleinsäuresequenz kodierend mindestens ein *Mycobacterium tuberculosis* (Mtb) Antigen; und
ii) eine zweite Nukleinsäuresequenz kodierend mindestens ein Mtb belebendes oder Mtb reaktivierendes Antigen, das nicht ein DosR Antigen ist.

2. Rekombinantes BCG nach Anspruch 1, wobei ein oder mehrere dieser mindestens zwei Nukleinsäuresequenzen in ein Chromosom des rBCG integriert ist.

3. Rekombinantes BCG nach Anspruch 1, wobei ein oder mehrere dieser mindestens zwei Nukleinsäuresequenzen eine extrachromosonmale Nukleinsäuresequenz ist.

4. Rekombinantes BCG nach Anspruch 1, wobei mindestens eines der Mtb Antigene, das 85a oder das mindestens eine Mtb Antigen das 85b ist.

5. Rekombinantes BCG nach Anspruch 1, wobei das mindestens eine Mtb Belebung- oder Reaktivierungsantigen ausgewählt ist aus der Gruppe bestehend aus Rv0867c, Rv1009, Rv1884c, Rv2389c, Rv2450c, Rv0288, Rv0685, Rv0824c, Rv2744c, Rv3347c, Rv1130 und Rv1169c.

6. Rekombinantes BCG nach Anspruch 1, wobei das mindestens *Mycobacterium tuberculosis* (Mtb) Antigen aus der Gruppe ausgewählt ist bestehend aus Rv2623, Rv2626c, Rv2005c, Rv1996, Rv2029c, Rv2627c, Rv1908, Rv3837, Rv2780 und Rv1349.

7. Rekombinantes BCG nach Anspruch 1, wobei die Expression des mindestens einen DosR Regulon Gens durch verstärkte Promotoraktivität heraufreguliert ist.

8. Rekombinantes Bazillus Calmette-Guerin (BCG), nach Anspruch 1 umfassend mindestens zwei Nukleinsäuresequenzen, die voneinander unterschiedlich sind und jedes hiervon kodiert ein oder mehrere Gene, die überexprimiert sind, wobei mindestens eine erste Nukleinsäuresequenz ausgewählt ist aus der Gruppe bestehend aus Rv2450c, Rv1009, Rv0867c, Rv2389c, Rv1884c, Rv0288, Rv0685, Rv0824c, Rv2744, Rv3347c, Rv1130, Rv1169c; und
wobei mindestens eine zweite Nukleinsäuresequenz ausgewählt ist aus der Gruppe bestehend aus Rv1738, Rv2623, Rv2031c, Rv2032, Rv2626c, Rv2005c, Rv3127, Rv1733c, Rv1996, Rv2628, Rv0079, Rv3130c, Rv3131, Rv1813c, Rv2006, Rv2029c, Rv2627c, Rv2030c, Rv2629, Rv2450c, Rv1009, Rv0867c, Rv2389c, Rv1884c, Rv0288, Rv0685, Rv0824c, Rv2744, Rv3347c, Ru1130, Rv1169c, Rv1886, Rv1980c, Rv3804c, Rv3875, Rv1926c, Rv0467, Rv3873, Rv1908c, Rv1174c, Rv2780, Rv2620c, Rv1793, Rv1349 und Rv3132; und wobei mindestens ein DosR Regulon Gen heraufreguliert ist.

9. Vakzine-Zusammensetzung umfassend ein rekombinantes Bazillus Calmette-Guerin (BCG) nach einem der Ansprüche 1 bis 8 zur Verwendung bei der Immunisierung eines Subjekts gegen eine Infektion mit Mtb oder zum Hervorrufen einer Immunantwort gegen Mtb in diesem Subjekt, wobei die Vakzine-Zusammensetzung verabreicht wird in einer Menge ausreichend zur Immunisierung des Subjekts gegen eine Infektion durch Mtb oder zum Hervorrufen einer Immunantwort gegen Mtb in diesem Subjekt.

10. Vakzine-Zusammensetzung zur Verwendung gemäß Anspruch 9, wobei die Vakzine-Zusammensetzung adaptiert ist an einer weiteren Verabreichung als Boost-Zusammensetzung umfassend ein oder mehrere Mtb Antigene.

11. Vakzine-Zusammensetzung zur Verwendung gemäß Anspruch 10, wobei das eine oder mehrere Antigene ausgewählt ist aus der Gruppe bestehend aus Rv1738, Rv2623, Rv2031c, Rv2032, Rv2626c, Rv2005c, Rv3127, Rv1733c, Rv1996, Rv2628, Rv0079, Rv3130c, Rv3131, Rv1813c, Rv2006, Rv2029c, Rv2627c, Rv2030c, Rv2629, Rv2450c, Rv1009, Rv0867c, Rv2389c, Rv1884c, Rv0288, Rv0685, Rv0824c, Rv2744, Rv3347c, Ru1130, Rv1169c, Rv1886, Rv1980c, Rv3804c, Rv3875, Rv1926c, Rv0467, Rv3873, Rv1908c, Rv1174c, Rv2780, Rv2620c, Rv1793, Rv1349 und Rv3132.

12. Vakzine-Zusammensetzung zur Verwendung nach Anspruch 10, wobei das eine oder mehrere Antigene ausgewählt ist aus der Gruppe bestehend aus Rv1996, Rv2005, Rv2029, Rv2623, Rv2626 und Rv2727 oder wobei das eine oder mehrere Antigene ausgewählt ist aus der Gruppe bestehend aus Rv2626, Rv1738, Rv2623, Rv1733, Rv2032, Rv3131, Rv3127, Rv3130c, Rv3804c und Rv1886c.

13. Vakzine-Zusammensetzung umfasst ein rekombinantes Bazillus Calmette-Guerin (BCG) gemäß einen der Ansprüche 1 bis 8 zur Verwendung in der Prävention eines Wiederauftretens von Symptomen einer Tuberkulose in einem Patienten mit einer latenten Mtb Infektion, wobei die Vakzine-Zusammensetzung verabreicht wird in einer Menge ausreichend, um ein Wiederauftreten von Symptomen der Tuberkulose in diesem Patienten zu verhindern.

14. Vakzine-Zusammensetzung zur Verwendung gemäß Anspruch 13 weiterhin umfassenden Schritt der Verabreichung einer Boost-Zusammensetzung umfassend Nukleinsäuresequenzen kodierend für ein oder mehrere Mtb Antigene.

15. Vakzine-Zusammensetzung zur Verwendung gemäß Anspruch 14, wobei das eine oder mehrere Antigene ausgewählt ist aus der Gruppe bestehend aus Rv1738, Rv2623, Rv2031c, Rv2032, Rv2626c, Rv2005c, Rv3127, Rv1733c, Rv1996, Rv2628, Rv0079, Rv3130c, Rv3131, Rv1813c, Rv2006, Rv2029c, Rv2627c, Rv2030c, Rv2629, Rv2450c, Rv1009, Rv0867c, Rv2389c, Rv1884c, Rv0288, Rv0685, Rv0824c, Rv2744, Rv3347c, Ru1130, Rv1169c, Rv1886, Rv1980c, Rv3804c, Rv3875, Rv1926c, Rv0467, Rv3873, Rv1908c, Rv1174c, Rv2780, Rv2620c, Rv1793, Rv1349 und Rv3132.

16. Vakzine-Zusammensetzung zur Verwendung gemäß Anspruch 14, wobei das eine oder mehrere Antigene ausgewählt ist aus der Gruppe bestehend aus Rv1996, Rv2005, Rv2029, Rv2623, Rv2626 und Rv2727, wobei das eine oder mehre Antigene ausgewählt ist aus der Gruppe bestehend aus Rv2626, Rv1738, Rv2623, Rv1733, Rv2032, Rv3131, Rv3127, Rv3130c, Rv3804c und Rv1886c.

17. Vakzine-Zusammensetzung zur Verwendung gemäß Anspruch 10, wobei diese einen oder mehrere Antigene mindestens ein Antigen aus jedem Stadium des Lebenszyklus von Mtb beinhalten.

## Revendications

1. Bacille de Calmette-Guérin (BCG) recombinant, dans lequel au moins un gène de régulon DosR est régulé à la hausse et ledit BCG comprend au moins deux séquences d'acide nucléique qui sont différentes l'une de l'autre et dont chacune code pour un ou plusieurs gène(s) qui est (sont) surexprimé(s), lesdites au moins deux séquences d'acide nucléique comprenant
i) une première séquence d'acide nucléique codant pour au moins un antigène de Mycobacterium tuberculosis (Mtb) ; et
ii) une deuxième séquence d'acide nucléique codant pour au moins un antigène de Mtb de ressuscitation ou de Mtb de réactivation, qui n'est pas un antigène DosR.

2. BCG recombinant selon la revendication 1, dans lequel une ou plusieurs desdites au moins deux séquences d'acide nucléique est (sont) intégrée(s) dans un chromosome dudit BCGr.

3. BCG recombinant selon la revendication 1, dans lequel une ou plusieurs desdites au moins deux séquences d'acide nucléique est (sont) une (des séquence(s) d'acide nucléique extra-chromosomique.

4. BCG recombinant selon la revendication 1, dans lequel ledit au moins un antigène de Mtb est un 85A ou ledit au moins un antigène de Mtb est un 85B.

5. BCG recombinant selon la revendication 1, dans lequel ledit au moins un antigène de Mtb de ressuscitation ou de réactivation est choisi dans le groupe constitué par les Rv0867c, Rv1009, Rv1884c, Rv2389c, Rv2450c, Rv0288, Rv0685, Rv0824c, Rv2744c, Rv3347c, Rv1130 et Rv1169c.

6. BCG recombinant selon la revendication 1, dans lequel ledit au moins un antigène de Mycobacterium tuberculosis (Mtb) est choisi dans le groupe constitué par les Rv2623, Rv2626c, Rv2005c, Rv1996, Rv2029c, Rv2627c, Rv1908, Rv3837, Rv2780 et Rv1349.

7. BCG recombinant selon la revendication 1, dans lequel l'expression dudit au moins un gène de régulon DosR est régulée à la hausse par une activité de promoteur renforcée.

8. Bacille de Calmette-Guérin (BCG) recombinant, selon la revendication 1, comprenant au moins deux séquences d'acide nucléique qui sont différentes l'une de l'autre et dont chacune code pour un ou plusieurs gène(s) qui est (sont) surexprimé(s),
dans lequel au moins une première séquence d'acide nucléique est choisie dans le groupe constitué par les Rv2450c, Rv1009, Rv0867c, Rv2389c, Rv1884c, Rv0288, Rv0685, Rv0824c, Rv2744, Rv3347c, Rv1130, Rv1169c ; et au moins une deuxième séquence d'acide nucléique est choisie dans le groupe constitué par les Rv1738, Rv2623, Rv2031c, Rv2032, Rv2626c, Rv2005c, Rv3127, Rv1733c, Rv1996, Rv2628, Rv0079, Rv3130c, Rv3131, Rv1813c, Rv2006, Rv2029c, Rv2627c, Rv2030c, Rv2629, Rv2450c, Rv1009, Rv0867c, Rv2389c, Rv1884c, Rv0288, Rv0685, Rv0824c, Rv2744, Rv3347c, Rv1130, Rv1169c, Rv1886, Rv1980c, Rv3804c, Rv3875, Rv1926c, Rv0467, Rv3873, Rv1908c, Rv1174c, Rv2780, Rv2620c, Rv1793, Rv1349 et Rv3132 ; et
dans lequel au moins un gène de régulon DosR est régulé à la hausse.

9. Composition vaccinale comprenant un Bacille de Calmette-Guérin (BCG) recombinant, selon l'une quelconque des revendications 1 à 8, destiné à être utilisé dans l'immunisation d'un sujet contre une infection par Mtb ou pour générer une réponse immunitaire contre un Mtb chez ledit sujet,
dans laquelle ladite composition vaccinale est administrée en une quantité suffisante pour immuniser ledit sujet contre une infection par Mtb ou pour générer une réponse immunitaire contre un Mtb chez ledit sujet.

10. Composition vaccinale, selon la revendication 9, destinée à être utilisée, selon la revendication 9, dans laquelle la composition vaccinale est adaptée à une administration supplémentaire en tant que composition de stimulation comprenant un ou plusieurs antigène(s) de Mtb.

11. Composition vaccinale, selon la revendication 10, destinée à être utilisée, selon la revendication 10, dans laquelle ledit un ou lesdits plusieurs antigène(s) est (sont) choisi(s) dans le groupe constitué par les Rv1738, Rv2623, Rv2031c, Rv2032, Rv2626c, Rv2005c, Rv3127, Rv1733c, Rv1996, Rv2628, Rv0079, Rv3130c, Rv3131, Rv1813c, Rv2006, Rv2029c, Rv2627c, Rv2030c, Rv2629, Rv2450c, Rv1009, Rv0867c, Rv2389c, Rv1884c, Rv0288, Rv0685, Rv0824c, Rv2744, Rv3347c, Rv1130, Rv1169c, Rv1886, Rv1980c, Rv3804c, Rv3875, Rv1926c, Rv0467, Rv3873, Rv1908c, Rv1174c, Rv2780, Rv2620c, Rv1793, Rv1349 et Rv3132.

12. Composition vaccinale, selon la revendication 10, destinée à être utilisée, selon la revendication 10, dans laquelle ledit un ou lesdits plusieurs antigène(s) est (sont) choisi(s) dans le groupe constitué par les Rv1996, Rv2005, Rv2029, Rv2623, Rv2626 et Rv2727 ou dans laquelle ledit un ou lesdits plusieurs antigène(s) est (sont) choisi(s) dans le groupe constitué par les Rv2626, Rv1738, Rv2623, Rv1733, Rv2032, Rv3131, Rv3127, Rv3130c, Rv3804c et Rv1886c.

13. Composition vaccinale comprenant un Bacille de Calmette-Guérin (BCG) recombinant, selon l'une quelconque des revendications 1 à 8, destiné à être utilisé dans la prévention de la récurrence des symptômes de la tuberculose chez un patient ayant une infection par Mtb latente,
dans laquelle ladite composition vaccinale est administrée en une quantité suffisante pour empêcher une récurrence des symptômes de la tuberculose chez ledit patient.

14. Composition vaccinale, selon la revendication 13, destinée à être utilisée, selon la revendication 13, comprenant en outre l'étape consistant à administrer une composition de stimulation, comprenant des séquences d'acide nucléique qui codent pour un ou plusieurs antigène(s) de Mtb.

15. Composition vaccinale, selon la revendication 14, destinée à être utilisée, selon la revendication 14, dans laquelle ledit un ou lesdits plusieurs antigène(s) est (sont) choisi(s) dans le groupe constitué par les Rv1738, Rv2623, Rv2031c, Rv2032, Rv2626c, Rv2005c, Rv3127, Rv1733c, Rv1996, Rv2628, Rv0079, Rv3130c, Rv3131, Rv1813c, Rv2006, Rv2029c, Rv2627c, Rv2030c, Rv2629, Rv2450c, Rv1009, Rv0867c, Rv2389c, Rv1884c, Rv0288, Rv0685, Rv0824c, Rv2744, Rv3347c, Rv1130, Rv1169c, Rv1886, Rv1980c, Rv3804c, Rv3875, Rv1926c, Rv0467, Rv3873, Rv1908c, Rv1174c, Rv2780, Rv2620c, Rv1793, Rv1349 et Rv3132.

16. Composition vaccinale, selon la revendication 14, destinée à être utilisée, selon la revendication 14, dans laquelle ledit un ou lesdits plusieurs antigène(s) est (sont) choisi(s) dans le groupe constitué par les Rv1996, Rv2005, Rv2029, Rv2623, Rv2626 et Rv2727 ou dans laquelle ledit un ou lesdits plusieurs antigène(s) est (sont) choisi(s) dans le groupe constitué par les Rv2626, Rv1738, Rv2623, Rv1733, Rv2032, Rv3131, Rv3127, Rv3130c, Rv3804c et Rv1886c.

17. Composition vaccinale, selon la revendication 10, destinée à être utilisée, selon la revendication 10, dans laquelle ledit un ou lesdits plusieurs antigène(s) comprend (comprennent) au moins un antigène provenant de chaque étape d'un cycle de vie du Mtb.
